# EUROPEAN PATENT SPECIFICATION

(11) **EP 2 759 595 B1**
(45) Date of publication and mention of the grant of the patent: **14.09.2016**
(21) Application number: 13305082.3
(22) Date of filing: 24.01.2013
(51) Int. Cl.: C12N 15/113, A61K 31/712

(54) **Composition comprising an encapsulated antagomir**
Zusammensetzung mit einem verkapselten Antagomir
Composition comprenant un antagomire encapsulé

(43) Date of publication of application: 30.07.2014
(73) Proprietor: Pierre Fabre Médicament S.A.S., 92654 Boulogne cedex (FR); Fundació Hospital Universitari Vall d'Hebron - Institut de Recerca, 08035 Barcelona (ES)
(72) Inventor: Asin, Miguel-Angel, 08290 Cerdanyola del Valles (ES); Ferret, Eulalia, 08820 El Prat de Llobregat (ES); Perez, Amadeo, 08028 Barcelona (ES); Garcia-Dorado Garcia, Antonio David, 08018 Barcelona (ES); Vert, Ignasi Barba, 08225 Terrassa (ES); Rodriguez Sinovas, Antonio, 08015 Barcelona (ES); Gotarda, Neus Bellera, 08027 Barcelona (ES)
(74) Representative: Nony

(56) References cited:
- BONAUER ANGELIKA ET AL: "MicroRNA-92a controls angiogenesis and functional recovery of ischemic tissues in mice", SCIENCE, AMERICAN ASSOCIATION FOR THE ADVANCEMENT OF SCIENCE, WASHINGTON, DC; US, vol. 324, no. 5935, 26 June 2009 (2009-06-26), pages 1710-1713, XP002575234, ISSN: 0036-8075
- D'ANGELO IVANA ET AL: "Improved delivery of angiogenesis inhibitors from PLGA:poloxamer blend micro- and nanoparticles.", JOURNAL OF MICROENCAPSULATION 2010, vol. 27, no. 1, 2010, pages 57-66, XP002700281, ISSN: 1464-5246
- PANYAM J ET AL: "Biodegradable nanoparticles for drug and gene delivery to cells and tissue", ADVANCED DRUG DELIVERY REVIEWS, ELSEVIER BV, AMSTERDAM, NL, vol. 55, no. 3, 24 February 2003 (2003-02-24), pages 329-347, XP008096954, ISSN: 0169-409X, DOI: 10.1016/S0169-409X(02)00228-4

### SEQUENCE LISTING

<110> PIERRE FABRE MEDICAMENT FUNDACIO HOSPITAL UNIVERSITARI VALL D'HERBRON-INSTITUT DE RECERCA
<120> Composition comprising an encapsulated antagomir
<130> BR96786-PFM
<160> 60
<170> PatentIn version 3.5
<210> 1
   <211> 78
   <212> RNA
   <213> Homo sapiens
<400> 1
   cuuucuacac agguugggau cgguugcaau gcuguguuuc uguaugguau ugcacuuguc      60
   ccggccuguu gaguuugg                                                    78
<210> 2
   <211> 75
   <212> RNA
   <213> Homo sapiens
<400> 2
   ucaucccugg guggggauuu guugcauuac uuguguucua uauaaaguau ugcacuuguc      60
   ccggccugug gaaga                                                       75
<210> 3
   <211> 96
   <212> RNA
   <213> Homo sapiens
<400> 3
   cgggccccgg gcgggcggga gggacgggac gcggugcagu guuguuuuuu cccccgccaa 60
   uauugcacuc gucccggccu ccggcccccc cggccc 96
<210> 4
   <211> 84
   <212> RNA
   <213> Homo sapiens
<400> 4
   gucagaauaa ugucaaagug cuuacagugc agguagugau augugcaucu acugcaguga 60
   aggcacuugu agcauuaugg ugac 84
<210> 5
   <211> 71
   <212> RNA
   <213> Homo sapiens
<400> 5
   ugcccuagca gcgggaacag uucugcagug agcgaucggu gcucuggggu auuguuuccg 60
   cugccagggu a 71
<210> 6
   <211> 89
   <212> RNA
   <213> Homo sapiens
<400> 6
   gucagcagug ccuuagcagc acguaaauau uggcguuaag auucuaaaau uaucuccagu 60
   auuaacugug cugcugaagu aagguugac 89
<210> 7
   <211> 81
   <212> RNA
   <213> Homo sapiens
<400> 7
   guuccacucu agcagcacgu aaauauuggc guagugaaau auauauuaaa caccaauauu        60
   acugugcugc uuuaguguga c                                                  81
<210> 8
   <211> 72
   <212> RNA
   <213> Homo sapiens
<400> 8
   uacaucggcc auuauaauac aaccugauaa guguuauagc acuuaucaga uuguauugua 60
   auugucugug ua 72
<210> 9
   <211> 72
   <212> RNA
   <213> Homo sapiens
<400> 9
   acucggaugg auauaauaca accugcuaag uguccuagca cuuagcaggu uguauuauca 60
   uuguccgugu cu 72
<210> 10
   <211> 70
   <212> RNA
   <213> Homo sapiens
<400> 10
   acacggacaa ugauaauaca accugcuaag ugcuaggaca cuuagcaggu uguauuauau 60
   ccauccgagu 70
<210> 11
   <211> 68
   <212> RNA
   <213> Homo sapiens
<400> 11
   cuccggugcc uacugagcug auaucaguuc ucauuuuaca cacuggcuca guucagcagg 60
   aacaggag 68
<210> 12
   <211> 73
   <212> RNA
   <213> Homo sapiens
<400> 12
   cucugccucc cgugccuacu gagcugaaac acaguugguu uguguacacu ggcucaguuc 60
   agcaggaaca ggg 73
<210> 13
   <211> 76
   <212> RNA
   <213> Homo sapiens
<400> 13
   gagggggaag acgggaggaa agaagggagu gguuccauca cgccuccuca cuccucuccu 60
   cccgucuucu ccucuc 76
<210> 14
   <211> 110
   <212> RNA
   <213> Homo sapiens
<400> 14
   ggccagcugu gaguguuucu uuggcagugu cuuagcuggu uguugugagc aauaguaagg 60
   aagcaaucag caaguauacu gcccuagaag ugcugcacgu uguggggccc 110
<210> 15
   <211> 84
   <212> RNA
   <213> Homo sapiens
<400> 15
   gugcucgguu uguaggcagu gucauuagcu gauuguacug uggugguuac aaucacuaac 60
   uccacugcca ucaaaacaag gcac 84
<210> 16
   <211> 77
   <212> RNA
   <213> Homo sapiens
<400> 16
   agucuaguua cuaggcagug uaguuagcug auugcuaaua guaccaauca cuaaccacac 60
   ggccagguaa aaagauu 77
<210> 17
   <211> 71
   <212> RNA
   <213> Homo sapiens
<400> 17
   guagcacuaa agugcuuaua gugcagguag uguuuaguua ucuacugcau uaugagcacu 60
   uaaaguacug c 71
<210> 18
   <211> 69
   <212> RNA
   <213> Homo sapiens
<400> 18
   aguaccaaag ugcucauagu gcagguaguu uuggcaugac ucuacuguag uaugggcacu 60
   uccaguacu 69
<210> 19
   <211> 83
   <212> RNA
   <213> Homo sapiens
<400> 19
   ccuuggagua aaguagcagc acauaauggu uuguggauuu ugaaaaggug caggccauau 60
   ugugcugccu caaaaauaca agg 83
<210> 20
   <211> 98
   <212> RNA
   <213> Homo sapiens
<400> 20
   uugaggccuu aaaguacugu agcagcacau caugguuuac augcuacagu caagaugcga 60
   aucauuauuu gcugcucuag aaauuuaagg aaauucau 98
<210> 21
   <211> 22
   <212> RNA
   <213> Homo sapiens
<400> 21
   uauugcacuu gucccggccu gu 22
<210> 22
   <211> 23
   <212> RNA
   <213> Homo sapiens
<400> 22
   agguugggau cgguugcaau gcu 23
<210> 23
   <211> 22
   <212> RNA
   <213> Homo sapiens
<400> 23
   ggguggggau uuguugcauu ac 22
<210> 24
   <211> 22
   <212> RNA
   <213> Homo sapiens
<400> 24
   uauugcacuc gucccggccu cc 22
<210> 25
   <211> 22
   <212> RNA
   <213> Homo sapiens
<400> 25
   agggacggga cgcggugcag ug 22
<210> 26
   <211> 22
   <212> RNA
   <213> Homo sapiens
<400> 26
   acugcaguga aggcacuugu ag 22
<210> 27
   <211> 23
   <212> RNA
   <213> Homo sapiens
<400> 27
   caaagugcuu acagugcagg uag 23
<210> 28
   <211> 23
   <212> RNA
   <213> Homo sapiens
<400> 28
   gggguauugu uuccgcugcc agg 23
<210> 29
   <211> 23
   <212> RNA
   <213> Homo sapiens
<400> 29
   uagcagcggg aacaguucug cag 23
<210> 30
   <211> 22
   <212> RNA
   <213> Homo sapiens
<400> 30
   ccaguauuaa cugugcugcu ga 22
<210> 31
   <211> 22
   <212> RNA
   <213> Homo sapiens
<400> 31
   ccaauauuac ugugcugcuu ua 22
<210> 32
   <211> 22
   <212> RNA
   <213> Homo sapiens
<400> 32
   uagcagcacg uaaauauugg cg 22
<210> 33
   <211> 22
   <212> RNA
   <213> Homo sapiens
<400> 33
   cuuaucagau uguauuguaa uu 22
<210> 34
   <211> 22
   <212> RNA
   <213> Homo sapiens
<400> 34
   uuauaauaca accugauaag ug 22
<210> 35
   <211> 22
   <212> RNA
   <213> Homo sapiens
<400> 35
   cuuagcaggu uguauuauca uu 22
<210> 36
   <211> 22
   <212> RNA
   <213> Homo sapiens
<400> 36
   auauaauaca accugcuaag ug 22
<210> 37
   <211> 22
   <212> RNA
   <213> Homo sapiens
<400> 37
   cacuuagcag guuguauuau au 22
<210> 38
   <211> 22
   <212> RNA
   <213> Homo sapiens
<400> 38
   auaauacaac cugcuaagug cu 22
<210> 39
   <211> 22
   <212> RNA
   <213> Homo sapiens
<400> 39
   uggcucaguu cagcaggaac ag 22
<210> 40
   <211> 22
   <212> RNA
   <213> Homo sapiens
<400> 40
   ugccuacuga gcugauauca gu 22
<210> 41
   <211> 22
   <212> RNA
   <213> Homo sapiens
<400> 41
   uggcucaguu cagcaggaac ag 22
<210> 42
   <211> 22
   <212> RNA
   <213> Homo sapiens
<400> 42
   ugccuacuga gcugaaacac ag 22
<210> 43
   <211> 21
   <212> RNA
   <213> Homo sapiens
<400> 43
   ucacuccucu ccucccgucu u 21
<210> 44
   <211> 22
   <212> RNA
   <213> Homo sapiens
<400> 44
   aagacgggag gaaagaaggg ag 22
<210> 45
   <211> 22
   <212> RNA
   <213> Homo sapiens
<400> 45
   caaucagcaa guauacugcc cu 22
<210> 46
   <211> 22
   <212> RNA
   <213> Homo sapiens
<400> 46
   uggcaguguc uuagcugguu gu 22
<210> 47
   <211> 22
   <212> RNA
   <213> Homo sapiens
<400> 47
   caaucacuaa cuccacugcc au 22
<210> 48
   <211> 23
   <212> RNA
   <213> Homo sapiens
<400> 48
   uaggcagugu cauuagcuga uug 23
<210> 49
   <211> 22
   <212> RNA
   <213> Homo sapiens
<400> 49
   aaucacuaac cacacggcca gg 22
<210> 50
   <211> 23
   <212> RNA
   <213> Homo sapiens
<400> 50
   aggcagugua guuagcugau ugc 23
<210> 51
   <211> 22
   <212> RNA
   <213> Homo sapiens
<400> 51
   acugcauuau gagcacuuaa ag 22
<210> 52
   <211> 23
   <212> RNA
   <213> Homo sapiens
<400> 52
   uaaagugcuu auagugcagg uag 23
<210> 53
   <211> 22
   <212> RNA
   <213> Homo sapiens
<400> 53
   acuguaguau gggcacuucc ag 22
<210> 54
   <211> 23
   <212> RNA
   <213> Homo sapiens
<400> 54
   caaagugcuc auagugcagg uag 23
<210> 55
   <211> 22
   <212> RNA
   <213> Homo sapiens
<400> 55
   caggccauau ugugcugccu ca 22
<210> 56
   <211> 22
   <212> RNA
   <213> Homo sapiens
<400> 56
   uagcagcaca uaaugguuug ug 22
<210> 57
   <211> 22
   <212> RNA
   <213> Homo sapiens
<400> 57
   cgaaucauua uuugcugcuc ua 22
<210> 58
   <211> 22
   <212> RNA
   <213> Homo sapiens
<400> 58
   uagcagcaca ucaugguuua ca 22
<210> 59
   <211> 16
   <212> DNA
   <213> artificial sequence
<220>
   <223> Antagomir
<400> 59
   ccgggacaag tgcaat 16
<210> 60
   <211> 16
   <212> RNA
   <213> artificial sequence
<220>
   <223> Antagomir
<400> 60
   ccgggacaag ugcaau 16

## Description

### Field of the invention

The present invention relates to the field of pharmaceutical formulations aimed at preventing or treating cardiac disorders, including cardiac ischemic disorders.

### Background of the invention

Acute myocardial infarction (AMI), also referred as myocardial infarction and commonly known as a heart attack, represents a major health risk in most industrialized nations throughout the world and remains a leading cause of morbidity and mortality worldwide.

Generally, AMI is caused by a sudden and sustained lack of blood flow to the heart tissue, which is usually the result of a narrowing or occlusion of a coronary artery. Without adequate blood supply, the tissue becomes ischemic, leading to the death of cardiomyocytes (heart muscle cells) and vascular structures.

There have been many advances in the treatment of AMI in recent decades, primarily in relation to coronary reperfusion in conjunction with pharmacological therapy. Reperfusion therapy has succeeded in changing the natural progression of AMI by reducing the infarcted area and improving short-term and long-term morbidity and mortality. However, there are substantial limitations to the use of the two reperfusion strategies currently available: fibrinolysis results in a low degree of coronary permeability and primary angioplasty cannot be applied frequently during the initial hours of evolution of the AMI. Furthermore, in some catheterized patients, the "no reflow" phenomenon or absence of microvascular reperfusion despite normal epicardial flow occurs, which results in adverse functional outcomes. This means that reperfusion therapy has not prevented the occurrence of deleterious remodelling of the myocardium, a complex intrinsic reparative process of collagen scar formation resulting in ventricular dilatation, contractile dysfunction and subsequent heart failure. Its occurrence, in approximately 30% of AMIs, has been mainly associated to higher infarction size, microvascular obstruction and unfavourable repair reactions still poorly understood.

Since reparative fibrosis and functional recovery of ischemic tissues is dependent on establishing networks that supply oxygenated blood, efforts have been made to improve vascular bed by inducing neoangiogenesis in the healing area for achieving direct conversion of injured areas to functional tissue *in situ.*

Therapeutic induction of angiogenesis could attenuate the occurrence of this phenomenon. Notwithstanding, the results of several years of clinical trials with intravenous proangiogenic factors have been unsatisfactory. Without being linked by a theory, it is considered that one of the reasons accounting for this failure may be that the intravenous route may not be able to reach and maintain an effective concentration of drug in the target tissue to promote and maintain a functional vascular network under severely compromised heart conditions.

In order to address this problem and prevent post-AMI cardiac insufficiency, research on the possibility of regenerating cardiac muscle and vessels was started within the last decade. Initial studies administrating pluripotent progenitor cells and infusing vascular growth factors showed promising results but the translation to the clinical setting showed inability of these therapies to regenerate neovasculature in an adequate manner for enabling destroyed cardiac muscle to recover.

Bonauer et al. ("MicroRNA-92a controls angiogenesis and functional recovery of ischemic tissues in mice"; Science; vol.324, no.5935, pages 1710-1713; 2009) teaches the involvement of miR-92a in angiogenesis and use of mir-92a inhibitor in mouse models of ischemia and myocardial infarction.

D'Angelo et al. ("Improved delivery of angiogenesis inhibitors from PLGA:poloxamer blend micro- and nanoparticles"; Journal of Microencapsulation; vol.27, no. 1, pages 57-66; 2010) teaches the delivery of angiogenesis inhibitors in microparticles.

At this day, the angiogenesis and repopulation of injured heart with cell therapy as well as specific drugs and resynchronization therapy can slow down the progression of this phenomenon but prevention of its occurrence remains a doubting challenge for cardiac medicine. Therefore, novel treatment strategies preventing adverse left ventricular remodelling are required.

### Summary of the invention

The present invention relates to the treatment of cardiac, preferably ischemic disorders by administering a composition that reverses or prevents ventricular remodelling by modulating the activity or expression of miR-92a. More particularly, the invention provides a composition comprising an inhibitor of miR-92a involved in angiogenesis, said inhibitor being preferably incorporated into a new delivery vehicle capable of releasing the inhibitor locally to the ischemic target tissue In addition, the invention provides compositions and kits useful for a method of reversing or preventing ventricular remodelling.

This invention relates to a composition comprising an effective amount of at least one inhibitor of a miRNA involved in the angiogenesis, wherein said inhibitor is microencapsulated into polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a.

In some embodiments of the composition described above, the said inhibitor of a miRNA is an oligonucleotide of 8-49 nucleotides in length having a sequence targeted to the said miRNA. In certain embodiments, the said oligonucleotide is an antisense oligonucleotide that is at least partially complementary to the sequence of the target miRNA. The said antisense oligonucleotide may be selected from the group consisting of a ribonucleotide, a deoxyribonucleotide, an antagomir, a LNA, a PNA, a morpholino oligonucleotide or a combination thereof.

In some embodiments of the composition described above, the said inhibitor of a miRNA consists of an antagomir, said antagomir comprising preferably a nucleotide sequence comprising at least 16 contiguous nucleotides complementary to the nucleotides of a sequence selected from the group consisting of SEQ ID No. 1, 2, 21, 22 and 23.

In some embodiments of the composition described above, the said inhibitor of a miRNA consists of an antagomir comprising the sequence SEQ ID No. 59 or 60 and modifications excluding base substitutions thereof, and fragments consisting of subsequences of SEQ ID NO: 59 or 60 of at least 8 contiguous nucleotides thereof.

In some embodiments of the composition described above, the said microspheres are presenting a diameter which does not exceed 25 µm, which encompasses microspheres having a diameter comprised between 5 and 15 µm.

In some embodiments of the composition described above, the said microspheres are made of a polymer consisting of poly-d,l-lactide (PLA), the said polymer being optionally blended with one or more other polymers.

This invention also relates to any composition as defined above for reversing or preventing ventricular remodelling in a subject in need thereof.

Thus, the invention also relates to any composition as defined above for reversing or preventing ventricular remodelling in a subject in need thereof, comprising administering to said subject an effective amount of said composition.

This invention also pertains to a population of biodegradable and biocompatible microspheres for use in the treatment or prevention of myocardial infarction, wherein said microspheres:
- have an average diameter comprised between 5 and 15 µm;
- are made of poly-d,l-lactide-co-glycolide (PLGA) ; poly-d,l-lactide (PLA) or a blend thereof;
- are incorporating from 1% to 15% w/w of a therapeutic agent capable of preventing ventricular remodelling,
wherein said therapeutic agent consists of an inhibitor of a miRNA selected from the group consisting of miR-92a.

### Description of the figures

**Figure 1** illustrates the morphology of antagomir-92a - PLGA microspheres determined by scanning electron microscopy;
**Figure 2** illustrates the size distribution of antagomir-92a - PLGA microspheres determined by laser light scattering;
**Figure 3** illustrates effects in hemodynamic and left ventricular contractibility by repeated injections of microspheres until 120mg;
**Figure 4** illustrates effects in hemodynamic and left ventricular contractibility by repeated injections of microspheres until 240mg;
**Figure 5** illustrates the protocol of the study of the molecular effect of a single intracoronary injection of microspheres according to the invention;
**Figure 6** illustrates the miR-92a inhibition specificity by the microspheres according to the invention;
**Figure 7** illustrates the encapsulated antagomir-92a induces angiogenesis in infarcted tissue. Vascular density in infarcted zone, calculated dividing the total number of vessels by the total infarcted area. N=20. * P< 0.01;

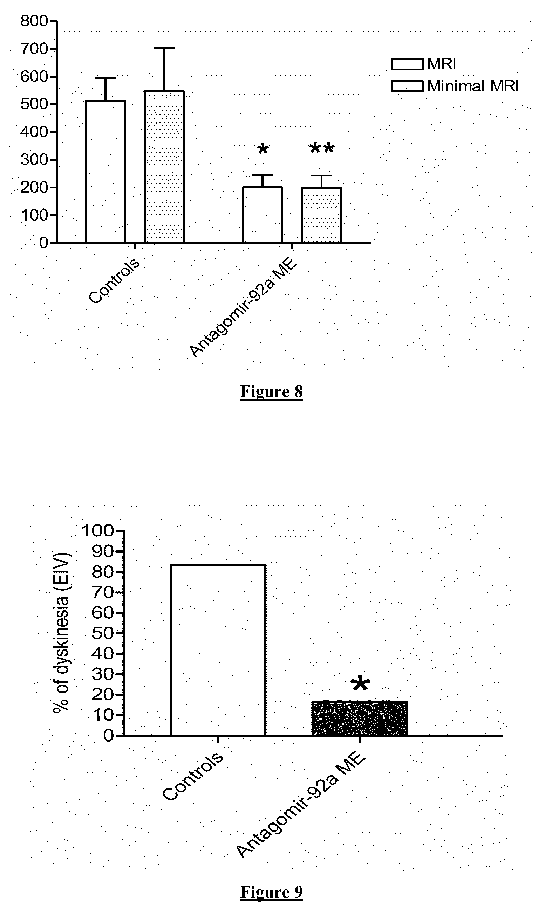

**Figure 8****:** Basal and minimal microvascular resistance index measured one month after AMI, in the infarct related artery of minipigs treated with encapsulated antagomir-92a, placebo or saline. **(a)** Basal microvascular resistance index (MRI) was calculated by multiplying pressure (mmHg) assessed with a pressure wire in positioned in the apical LAD (Pd) and coronary flow (ml/min) quantified by a sensor positioned in the distal segment of LAD. Minimal microvascular resistance index (MRI_{hyp}) was calculated with the same parameters measured in maximal hyperemia achieved with intravenous infusion of 140 micro/Kg/min of adenosine administered through the femoral vein 12F sheath. n= 12 * p<0.01 ** p=0.05 **(b)** Correlation between the total number of vessels in the necrotic area and MRI. R² 0.41, p=0.02, n=12. (c) Correlation between the total number of vessels in the necrotic area and minimal MRI. R² 0.27, p=0.08, n=12;
**Figure 9****:** One month after AMI, the presence of septoapical diskynesia was avaluated by an echocardiographer blind to the allocated treatment. The percentage of animals with septoapicaldiskynesia in treated and non-treated animals is shown (83.3% vs 16.7%, p=0.03). n=20.

### Detailed Description of the invention

Some definitions are given hereunder that are relevant as regards the description of the whole embodiments that are encompassed by the present invention.

MicroRNAs (miRs) are small, noncoding RNAs that are emerging as crucial regulators of biological processes.

"MicroRNA", "miRNA" or "miR" means a non coding RNA of about 18 to about 25 nucleotides in length. These miRs could originate from multiple origins including: an individual gene encoding for a miRNA, from introns of protein coding gene, or from polycistronic transcript that often encode multiple, closely related microRNAs.

Current knowledge shows that miRNAs are transcribed by RNA polymerase II (pol II) or RNA polymerase III (pol III) and arise from initial transcripts, termed primary miRNA transcripts (pri-miRNAs), that are generally several thousand bases long. Pri-miRNAs are processed in the nucleus by the RNase Drosha into about 70 to 100-nucleotide hairpin-shaped precursors (pre-miRNAs). Following transport to the cytoplasm, the hairpin pre-miRNA is further processed by Dicer to produce a double-stranded microRNA; one of the strands, the so called mature microRNA, (sometimes both strands can be used) is then incorporated into the RNA-induced silencing complex (RISC), where it associates with its target mRNAs by base-pair complementarity. In the relatively rare cases in which a miRNA base pairs perfectly with a messenger RNA (mRNA) target, it promotes mRNA degradation. More commonly, microRNAs form imperfect heteroduplexes with target mRNAs, affecting either mRNA stability or inhibiting mRNA translation.

"Stem-loop sequence" means a RNA having a hairpin structure and containing a mature microRNA sequence. Pre-miRNA sequences and stem-loop sequences may overlap. Examples of stem-loop sequences are found in the microRNA database known as miRBase.

"microRNA precursor" means a transcript that originates from a genomic DNA and that comprises a non-coding, structured RNA comprising one or more microRNA sequences. For example, in certain embodiments a microRNA precursor is a pre-miRNA. A microRNA precursor may be a pri-miRNA.

The following specification will follow the standard nomenclature system with the uncapitalized "mir-X" refers to the pre-miRNA, while a capitalized "miR-X" refers to the mature form. When two mature microRNAs originate from opposite arms of the same pre-miRNA, they are denoted with a -3p or -5p suffix. When relative expression levels are known, an asterisk following the name indicates a microRNA expressed at low levels relative to the microRNA in the opposite arm of a hairpin.

In the following specification, unless otherwise specified, the use of the expression miR-X refers to the mature miRNA including both forms -3p and -5p, if any.

For the avoidance of doubt, in the present specification, the expressions microRNA, miRNA and miR designate the same product.

In the context of the present invention, it is an objective to modulate angiogenesis or angiogenic processes with, as a consequence, a particular focus on miR-92a involved in angiogenesis. It is thus an objective of the invention to modulate at least miR-92a belonging to a family of microRNAs involved in the modulation of the angiogenesis.

By the expression "microRNA family", it is intended a group of microRNAs with a related function consisting of the modulation of angiogenesis or angiogenic processes. More particularly, without limitation, said microRNAs are selected in the group consisting of microRNAs presenting an antiangiogenic activity.

For the avoidance of doubt, the expression microRNA in the following specification, unless otherwise indicated, shall refer to the mature or processed RNA after it has been cleaved from its precursor. For the non mature forms, the expression "precursors" or "microRNA precursors" will be used.

The following table 1 regroups different sequences of microRNA precursors.

**Table 1**

| **SEQ ID NO:** | **Name** | **Sequence** |
|---|---|---|
| 1 | mir-92a-1 | |
| 2 | mir-92a-2 | |
| 3 | mir-92b | |
| 4 | mir-17 | |
| 5 | mir-503 | |
| 6 | mir-16-1 | |
| 7 | mir-16-2 | |
| 8 | mir-374a | |
| 9 | mir-374b | |
| 10 | mir-374c | |
| 11 | mir-24-1 | |
| 12 | mir-24-2 | |
| 13 | mir-483 | |
| 14 | mir-34a | |
| 15 | mir-34b | |
| 16 | mir-34c | |
| 17 | mir-20a | |
| 18 | mir-20b | |
| 19 | mir-15a | |
| 20 | mir-15b | |

The following table 2 indicates, for each microRNA, the sequences of the microRNA and the corresponding residues into the corresponding precursor sequences (see table 1).

**Table 2**

| **SEQ ID NO:** | **Name** | **Sequence** | **Residues from precursor** |
|---|---|---|---|
| 21 | miR-92a-3p | uauugcacuugucccggccugu | 48-69 |
| 22 | miR-92a-1-5p | agguugggaucgguugcaaugcu | 11-33 |
| 23 | miR-92a-2-5p | ggguggggauuuguugcauuac | 9-30 |
| 24 | miR-92b-3p | uauugcacucguaccggccucc | 61-82 |
| 25 | miR-92b-5p | agggacgggacgcggugcagug | 20-41 |
| 26 | miR-17-3p | acugcagugaaggcacuuguag | 51-72 |
| 27 | miR-17-5p | caaagugcuuacagugcagguag | 14-36 |
| 28 | miR-503-3p | gggguauuguuuccgcugccagg | 46-68 |
| 29 | miR-503-5p | uagcagcgggaacaguucugcag | 6-28 |
| 30 | miR-16-1-3p | ccaguauuaacugugcugcuga | 56-77 |
| 31 | miR-16-2-3p | ccaauauuacugugcugcuuua | 53-74 |
| 32 | miR-16-5p | uagcagcacguaaauauuggcg | 14-35 (for miR-16-1) or 10-31 (for miR-16-2) |
| 33 | miR-374a-3p | cuuaucagauuguauuguaauu | 42-63 |
| 34 | miR-374a-5p | uuauaauacaaccugauaagug | 12-33 |
| 35 | miR-374b-3p | cuuagcagguuguauuaucauu | 41-62 |
| 36 | miR-374-5p | auauaauacaaccugcuaagug | 11-32 |
| 37 | miR-374c-3p | cacuuagcagguuguauuauau | 39-60 |
| 38 | miR-374c-5p | auaauacaaccugcuaagugcu | 13-34 |
| 39 | miR-24-1-3p | uggcucaguucagcaggaacag | 44-65 |
| 40 | miR-24-1-5p | ugccuacugagcugauaucagu | 7-28 |
| 41 | miR-24-2-3p | uggcucaguucagcaggaacag | 50-71 |
| 42 | miR-24-2-5p | ugccuacugagcugaaacacag | 13-34 |
| 43 | miR-483-3p | ucacuccucuccucccgucuu | 48-68 |
| 44 | miR-483-5p | aagacgggaggaaagaagggag | 8-29 |
| 45 | miR-34a-3p | caaucagcaaguauacugcccu | 64-85 |
| 46 | miR-34a-5p | uggcagugucuuagcugguugu | 22-43 |
| 47 | miR-34b-3p | caaucacuaacuccacugccau | 50-71 |
| 48 | miR-34b-5p | uaggcagugucauuagcugauug | 13-35 |
| 49 | miR-34c-3p | aaucacuaaccacacggccagg | 46-67 |
| 50 | miR-34c-5p | aggcaguguaguuagcugauugc | 13-35 |
| 51 | miR-20a-3p | acugcauuaugagcacuuaaag | 44-65 |
| 52 | miR-20a-5p | uaaagugcuuauagugcagguag | 8-30 |
| 53 | miR-20b-3p | acuguaguaugggcacuuccag | 44-65 |
| 54 | miR-20b-5p | caaagugcucauagugcagguag | 6-28 |
| 55 | miR-15a-3p | caggccauauugugcugccuca | 51-72 |
| 56 | miR-15a-5p | uagcagcacauaaugguuugug | 14-35 |
| 57 | miR-15b-3p | cgaaucauuauuugcugcucua | 58-79 |
| 58 | miR-15b-5p | uagcagcacaucaugguuuaca | 20-41 |

Unless otherwise indicated, precursor and microRNA sequences referred to in the application are human sequences. Nevertheless, in some cases, microRNA human sequences are homologous to microRNA sequences from other species.

As an example, it can be mentioned here that the human miR-92a is homologous to miRs from other species. More particularly, the human miR-92a is homologous to miRs from dme (*Drosophila melanogaster*), mmu (*Mus musculus*), rno (*Rattus norvegicus*), dps (*Drosophila pseudoobscura*), aga (*Anopheles gambiae*), dre (*Danio rerio*), mml (*Macaca mulatta*), xtr (*Xenopus tropicalis*), ame (*Apis mellifera),* odi (*Oikopleura dioica*), cin (*Ciona intestinalis),* csa (*Ciona savignyi),* cfa (*Canis familiaris)* and pig or ssc (*Sus scrofa*).

Based on its general knowledge, the person skilled in the art will find easily the homology between other human microRNA sequences and microRNA sequences from other species.

Nucleotide sequences of mature microRNAs and their corresponding stem-loop sequences described herein are the sequences found in miRBase, an online searchable database of microRNA sequences and annotation. Entries in the miRBase Sequence database represent a predicted hairpin portion of a microRNA transcript (the stem-loop), with information on the location and sequence of the mature microRNA sequence. The microRNA stem-loop sequences in the database are not strictly precursor miRNAs (pre-miRNAs), and may in some instances include the pre-miRNA and some flanking sequence from the presumed primary transcript.

It is thus an objective of the present invention to provide compositions for the treatment or prevention of ventricular remodelling after AMI comprising an inhibitor of miR-92a, or a combination of inhibitors of miR-92a, involved in different reactions governing the physiology of angiogenesis, or precursors thereof.

However, in most of the published studies, the intravenous route is used for administering microRNA inhibitors in animals. The manipulation of microRNA involved in the regulation of vascular genes expression represents a novel therapeutic target in ischaemic disease. Within the biomedical sector, there has been an exponential increase in research on the administration of RNA modulators especially designed to inhibit a particular RNA sequence.

Dimmeler et al have shown improvement in contractility and recovery of left ventricular function after miR-92a inhibition by specific microRNA inhibitor administered systemically. Since the polycistronic microRNA 17-92a cluster has been linked to tumorigenesis and because of microRNAs cellular type ubiquity, intravenous administration in repetitive injections of miR or anti-miRs raises concerns about safety.

Owing to the fact that microRNAs control complex processes and are present in various cellular pathways, it is highly probable that they also trigger significant side effects. An issue, among other ones, at present is that treatments with miRNA inhibitors are not selective.

In addition, given the ubiquitousness and low organ specificity of these molecules, systemic administration could lead to exercise regulatory functions in tissues where these miRNAs have different cell-specific functions or where are not normally expressed. This erroneous regulation would likely lead to triggering side effects. Among the potential risks, tumorigenesis associated with microRNA manipulation remains one of the primary concerns when applying this therapy to human pathology. Moreover, in order to obtain an adequate sustained concentration in the target cells, microRNA inhibitors must be repeatedly injected at very high doses. Although experimentation on small animals in controlled conditions carries minor limitations, the transposition to human beings presupposes obstacles to biosafety as well as logistical and economic hindrances.

In order to solve these problems and enable this new therapeutic approach to be transferred to patients, it is there considered to generate a release vehicle for transporting microRNA inhibitors and releasing them directly onto the target organ. An appropriate vehicle would direct the microRNA inhibitor straight to the diseased tissue thereby enabling a reduction in dosage, so as to avoid the administration of repeated injections and minimise potentially undesirable biological effects on other organs.

The objective is therefore to improve selectivity in order to avoid the side effects of these treatments by means of releasing the microRNA inhibitors only in target tissue. This issue is solved, according to the invention, by microencapsulating the microRNA inhibitors into biodegradable and biocompatible microspheres.

It is thus an objective of the present invention to provide compositions for the treatment or prevention of ventricular remodelling after AMI comprising at least one microencapsulated inhibitor of miR-92a belonging to the microRNA family related to the modulation of angiogenesis.

By microencapsulating microRNA inhibitors in microspheres, intra-arterial administration after transluminal angioplasty is facilitated, so that the microspheres are delivered and retained only in the microvessels, also referred to as capillaries, of the damaged area; in this way the encapsulated microRNA inhibitors can be released locally.

Intracoronary injection through the culprit artery of AMI enables retention of the microspheres in the coronary microcirculation and sustained release of the microRNA inhibitors directly in the target ischemic tissue. The microRNA inhibitors induce neoangiogenesis which enhances functional recovery of the contractility of the damaged tissue as well as favourable post-infarction remodelling.

Unexpectedly, it is shown herein that, when it is microencapsulated into appropriate microspheres, a microRNA inhibitor and especially an inhibitor of miR-92a belonging to the microRNA family related to the modulation of angiogenesis, the said microRNA is successfully released to the damaged area of the myocardium so as to block the biological activity of the target microRNA. As shown in the examples herein, the administration, by selective intracoronary route, of an inhibitor of miR-92a, that is microencapsulated into polymeric biodegradable and biocompatible microspheres to individuals having undergone a myocardial infarction leads to myocardial functional recovery.

Therefore, microspheres retained in microcirculation allow sustained release of the microRNA inhibitor directly in the target ischemic tissue. The sustained effect of microRNA inhibitors (also known as down-regulation) of targeted microRNA resulted in a significant vessel growth and suppression of adverse remodeling in the healing area one month after injury.

As shown in the examples herein, it has been demonstrated that the occurrence of adverse ventricular remodelling can be prevented by inducing vasculogenesis through local and sustained inhibition of miR-92a belonging to the microRNA family related to the modulation of angiogenesis by encapsulated appropriate microRNA inhibitor administered in the infarcted related artery. This represents a new delivery method that will facilitate the next safety translation of gene modulation therapy to patients that suffer an acute myocardial infarction.

These results clearly show that a composition comprising an inhibitor of miR-92a consisting of an antisense oligonucleotide targeting or being complementary to miR-92a, as disclosed herein, allows an effective local release of the said inhibitor in therapeutically effective amounts and at a therapeutically effective release rate.

In a first aspect, the invention relates to a composition comprising an effective amount of at least one inhibitor of a microRNA involved in the angiogenesiswherein said inhibitor is microencapsulated into polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a.

By the expression "microspheres", it must be understood spherical particles sized from 1 µm to few hundred µm, which includes a diameter comprised between 5 and 15 µm. The expression "microparticles" includes both "microspheres" and "microcapsules". In the present specification, the expression "microspheres" is used but it must be understood that, when the substitution with a "microcapsule" is possible and of interest according to the man skilled in the art, the use of "microspheres" and "microcapsules" is equivalent.

By the expression "encapsulated" or "microencapsulated", it must be understood enclosed or embedded in particles for protection or for modified release.

In other words, the invention relates to a pharmaceutical composition comprising an effective amount of at least one inhibitor of a microRNA involved in angiogenesis, wherein said inhibitor is microencapsulated in polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a.

"Pharmaceutical composition" means a mixture of substances suitable for administering to an individual that includes a pharmaceutical agent. For example, a pharmaceutical composition may comprise a miRNA inhibitor and a sterile aqueous solution.

In an embodiment of the composition of the invention, the said microRNA consists of the mature miR-92a comprising a sequence selected from the group consisting of SEQ ID No. 21, 22 or 23 or a sequence having at least 90% nucleotide identity with one of SEQ ID No. 21, 22 or 23.

In another embodiment of the composition of the invention, the said miR-92a consists of the mature miR-92a comprising the sequence SEQ ID No. 21 or a sequence having at least 90%, preferably 95%, nucleotide identity with SEQ ID NO 21.

In another embodiment of the composition of the invention, the said miR-92a consists of the mature miR-92a comprising the sequence SEQ ID No. 22 or a sequence having at least 90%, preferably 95% nucleotide identity with SEQ ID NO 22.

In another embodiment of the composition of the invention, the said miR-92a consists of the mature miR-92a comprising the sequence SEQ ID No. 23 or a sequence having at least 90%, preferably 95%, nucleotide identity with SEQ ID NO 23.

In the sense of the present invention, the "percentage identity" between two sequences of nucleic acids means the percentage of identical nucleotides residues between the two sequences to be compared, obtained after optimal alignment, this percentage being purely statistical and the differences between the two sequences being distributed randomly along their length. The comparison of two nucleic acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970, by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

The percentage identity between two nucleic acid sequences is determined by comparing the two optimally-aligned sequences in which the nucleic acid sequence to compare can have additions or deletions compared to the reference sequence for optimal alignment between the two sequences. Percentage identity is calculated by determining the number of positions at which the nucleotide residue is identical between the two sequences, preferably between the two complete sequences, dividing the number of identical positions by the total number of positions in the alignment window and multiplying the result by 100 to obtain the percentage identity between the two sequences.

As intended herein, nucleotide sequences having at least 90% nucleotide identity with a reference sequence encompass those having at least 91%, 92%, 93%, 94%, 95%, 96%, 97%, 98% and 99% nucleotide identity with the said reference sequence.

Generally speaking, an inhibitor is a molecule which represses or prevents another molecule from engaging in a reaction.

As used herein, the term "inhibitor of miR-X, or mir-X" refers to any molecule or compound that decreases or reduces the expression and/or activity of miR-X, or mir-X. This inhibition should, as a consequence, prevent neo-angiogenesis inhibition, i.e. promote neoangiogenesis so as to prevent the adverse remodelling of cardiac muscle after the infarction.

In one embodiment of the invention, the said inhibitor of miR-92a is an oligonucleotide of 8-49 nucleotides in length having a sequence targeted to said miR-92a.

The expression "Targeted to" means having a nucleotide sequence that will allow hybridization to a target nucleic acid to induce a desired effect. In certain embodiments, a desired effect is reduction and/or inhibition of a target nucleic acid.

"Hybridize" means the annealing of complementary nucleic acids that occurs through "nucleotide complementarity", i.e. the ability of two nucleotides to pair non-covalently via hydrogen bonding.

On some embodiments, miRNA inhibitor oligonucleotides are 8 to 49 nucleotides in length.

One having ordinary skill in the art will appreciate that this embodies oligonucleotides of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides in length, or any range within. In some embodiments, oligonucleotides according to the invention, are 10 to 20 nucleotides in length. One having ordinary skill in the art will appreciate that this embodies oligonucleotides of 10, 11, 12, 13, 14, 15, 16, 17, 18, 19 or 20 nucleotides in length, or any range within.

In certain embodiments, the oligonucleotide has a sequence that is complementary to miR-92a.

In one embodiment of the composition of the invention, the said oligonucleotide is an antisense oligonucleotide that is at least partially complementary to the sequence of miR-92a.

The expression "antisense oligonucleotide" refers to an oligonucleotide having a nucleotide sequence complementary to a specific nucleotide sequence (referred to as a sense sequence) and capable of hybridizing with the sense sequence.

"Complementarity" means the nucleotide pairing ability between a first nucleic acid and a second nucleic acid.

In certain embodiments, an antisense oligonucleotide has a nucleotide sequence that is complementary to miR-92a, meaning that the sequence of the antisense oligonucleotide is at least 60%, 65%, 70%, 75%, 80%, 85%, 90%, 95%, 97%, 98% or 99% identical to the complement of miR-92a, or that the two sequences hybridize under stringent hybridization conditions. Accordingly, in certain embodiments the nucleotide sequence of the antisense oligonucleotide may have one or more mismatched basepairs with respect to miR-92a, and is capable of hybridizing to its target sequence. In certain embodiments, the antisense oligonucleotide has a sequence that is fully complementary to miR-92a, meaning that the nucleotide sequence of the antisense oligonucleotide is 100% identical of the complement of miR-92a.

In the context of the present invention, "Complementary" means an antisense oligonucleotide having a nucleotide sequence that is at least 60%, at least 65%, at least 70%, at least 75%, at least 80%, at least 85%, at least 90%, at least 95%, at least 97%, at least 98% at least 99%, or 100%, identical to the complement of the nucleotide sequence of miR-92a, over a region of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides, or that the two sequences hybridize under stringent hybridization conditions.

"Percent complementarity" means the number of complementary nucleotide in a nucleic acid divided by the length of the nucleic acid. In certain embodiments, percent complementarity of an oligonucleotide means the number of nucleotides that are complementary to the target nucleic acid, divided by the length of the oligonucleotide.

In one embodiment, the antisense oligonucleotide sequence is "fully complementary" to the sequence of the target miR-92a, which means that each nucleotide of the antisense oligonucleotide is capable of pairing with a nucleotide at each corresponding position in the target microRNA.

In certain embodiment, the antisense oligonucleotide according to the invention has a sequence that is partially or fully complementary to the sequence of miR-92a comprising a sequence selected from the group consisting of SEQ ID No. 21, 22 or 23 or a sequence having at least 90% nucleotide identity with one of SEQ ID No. 21, 22 or 23.

In one embodiment, the antisense oligonucleotide comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones.

Morpholino oligonucleotides have an uncharged backbone in which the deoxyribose sugar of DNA is replaced by a six membered ring and the phosphodiester linkage is replaced by a phosphorodiamidate linkage. Morpholino oligonucleotides are resistant to enzymatic degradation and appear to function as antisense agents by arresting translation or interfering with pre-mRNA splicing rather than by activating RNase H.

A modified backbone is typically preferred to increase nuclease resistance. A modified backbone can also be preferred because of its altered affinity for the target sequence compared to an unmodified backbone. An unmodified backbone can be RNA or DNA.

Another suitable antisense oligonucleotide comprises a Peptide Nucleic Acid (PNA), having a modified polyamide backbone. PNA-based molecules are true mimics of DNA molecules in terms of base-pair recognition. The backbone of the PNA is composed of 7V-(2-aminoethyl)- glycine units linked by peptide bonds, wherein the nucleobases are linked to the backbone by methylene carbonyl bonds.

A further suitable backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.

In yet a further embodiment, an antisense oligonucleotide of the invention comprises a substitution of one of the non-bridging oxygens in the phosphodiester linkage. This modification slightly destabilizes base- pairing but adds significant resistance to nuclease degradation.

A further suitable antisense oligonucleotide of the invention comprises one or more sugar moieties that are mono- or disubstituted at the 2', 3' and/or 5' position such as a -OH; -F; substituted or unsubstituted, linear or branched lower (CI- CIO) alkyl, alkenyl, alkynyl, alkaryl, allyl, aryl, or aralkyl, that may be interrupted by one or more heteroatoms; 0-, S-, or N-alkyl; 0-, S-, or N-alkenyl; 0-, S-or N-alkynyl; 0-, S-, or N-allyl; O-alkyl-O-alkyl, -methoxy, -aminopropoxy; -amino xy; methoxyethoxy; - dimethylaminooxyethoxy; and -dimethylaminoethoxyethoxy.

The sugar moiety can be a pyranose or derivative thereof, or a deoxypyranose or derivative thereof, preferably a ribose or a derivative thereof, or a deoxyribose or a derivative thereof. Such preferred derivatized sugar moieties comprise Locked Nucleic Acid.

An LNA is a modified RNA nucleotide wherein the ribose moiety of LNA nucleotide is modified with an extra bridge connecting 2' and 4' carbons. This enhances the base stacking and pre-organization, and significantly increases the thermal stability. This bridge "locks" the ribose in 3'-endo structural conformation, which is often found in A-form of DNA or RNA. LNA nucleotides used in the present invention can be mixed with DNA or RNA bases in the oligonucleotide whenever desired.

According to the invention, the said antisense oligonucleotide is selected in the group consisting of a ribonucleotide, a deoxyribonucleotide, an antagomir, a LNA, a PNA, a morpholino oligonucleotide or a combination thereof.

In a preferred embodiment of the composition of the invention, the said antisense oligonucleotide is an antagomir.

Antagomirs are chemically engineered oligonucleotides which are used to silence endogenous microRNA. An antagomir is a small synthetic RNA or DNA that is perfectly complementary to the specific microRNA target with either mispairing at the cleavage site or some sort of base modification to inhibit cleavage. Usually, antagomirs have some sort of modification to make it more resistant to degradation and facilitate cellular internalization. It is unclear how antagomirization (the process by which an antagomir inhibits microRNA activity) operates, but it is believed to inhibit by irreversibly binding the microRNA. Antagomirs are used to constitutively inhibit the activity of specific microRNAs.

In an embodiment of the invention, the said antagomir comprises a nucleotide sequence comprising at least 8, 9, 10, 11, 12, 13, 14, 15 or 16 contiguous nucleotides complementary to the mir-92a of sequence selected from the group consisting of SEQ ID No. 1 and 2.

In an embodiment of the invention, the said antagomir comprises a nucleotide sequence comprising at least 8, 9, 10, 11, 12, 13, 14, 15 or 16 contiguous nucleotides complementary to the miR-92a of sequence selected from the group consisting of SEQ ID No. 21, 22 or 23.

In another embodiment, the said antagomir comprises a nucleotide sequence comprising at least 16 contiguous nucleotides complementary to the nucleotides of sequence SEQ ID No. 21.

In an embodiment of the invention, the said antagomir possesses a DNA backbone.

In said embodiment of the composition of the invention, the said antagomir comprises the sequence SEQ ID No. 59 and modifications excluding base substitutions thereof, and fragments consisting of subsequences of SEQ ID NO: 59 of at least 8 contiguous nucleotides thereof.

In another embodiment of the invention, the said antagomir possesses a RNA backbone.

In said embodiment of the composition of the invention, the said antagomir comprises the sequence SEQ ID No. 60 and modifications excluding base substitutions thereof, and fragments consisting of subsequences of SEQ ID NO: 60 of at least 8 contiguous nucleotides thereof.

In one embodiment, the antagomir according to the invention is a fragment consisting of a subsequence of SEQ ID NO: 59 or 60 of at least 8 contiguous nucleotides thereof.

In one embodiment, the antagomir according to the invention is a fragment consisting of a subsequence of SEQ ID NO: 59 or 60 of at least 9 contiguous nucleotides thereof.

In one embodiment, the antagomir according to the invention is a fragment consisting of a subsequence of SEQ ID NO: 59 or 60 of at least 10 contiguous nucleotides thereof.

In one embodiment, the antagomir according to the invention is a fragment consisting of a subsequence of SEQ ID NO: 59 or 60 of at least 11 contiguous nucleotides thereof.

In one embodiment, the antagomir according to the invention is a fragment consisting of a subsequence of SEQ ID NO: 59 or 60 of at least 12 contiguous nucleotides thereof.

In one embodiment, the antagomir according to the invention is a fragment consisting of a subsequence of SEQ ID NO: 59 or 60 of at least 13 contiguous nucleotides thereof.

In one embodiment, the antagomir according to the invention is a fragment consisting of a subsequence of SEQ ID NO: 59 or 60 of at least 14 contiguous nucleotides thereof.

In one embodiment, the antagomir according to the invention is a fragment consisting of a subsequence of SEQ ID NO: 59 or 60 of at least 15 contiguous nucleotides thereof.

In another embodiment, the said antagomir of sequence SEQ ID No. 59 or 60 presents at least 1, 2, 3, 4, 5, 6 or 7 modified nucleotide(s) by phosphotioate bond(s) between adjacent nucleotide.

In other embodiment of the invention, the said antagomir can include 2'-O-methyl modified nucleotide, cholesterol group or any similar or equivalent modification.

Generally, if a pharmaceutical formulation containing a nucleic acid, a peptide and a protein is administered orally or parenterally, it is degraded by enzymes in the body, and the efficacy of the pharmaceutical formulation disappears quickly. Various trials have been made to conquer the problem. One of which is to formulate a long sustained-release injectable.

In practice, research is being conducted on the use of stents as a system for the selective administration of microRNAs. However, rapid endothelialization of the stents could pose a problem with regard to the sustained release of microRNAs, especially for biological processes requiring prolonged gene modulation. In addition, liposomes and nanoparticles have been developed and used in vivo but pass to the circulatory system with risk of serious side effects have not been avoided. In addition, no studies have demonstrated efficacy and safety after percutaneous intracoronary administration without previous aortic clamp.

Another aspect of the invention is based on the use of biodegradable and biocompatible microspheres.

According to the invention, it has been designed and manufactured a release system appropriate for localised release of oligonucleotides in the cardiac region, based on the microencapsulation of said oligonucleotides with biocompatible biodegradable polymers. The invention allows to obtain microspheres substantially loaded with oligonucleotides, which have high encapsulation efficiency and no molecule modification/degradation. According to the invention, the purity and quality of the molecule, without adding either a stabilising agent or a retention substance, is preserved, thanks to the manufacturing conditions used, particularly the characteristics of the emulsion created, the concentration of the polymer solution and the relation between the volumes of the phases involved in the microencapsulation process. In addition, the microspheres have an appropriate particle-size distribution to enable them to be retained in the microvessels of the infarcted area without causing arterial emboliation and without being destroyed by macrophage phagocytic action.

An object of the present invention is to provide a sustained-release microsphere, which stably encapsulates a short chain deoxyribonucleic acid or a short chain ribonucleic acid, and is able to inhibit, for a long period, the inhibition of the expression of a specific protein, especially a protein whose inhibition is related to a disease.

Generally speaking, "biocompatible" means compatible with living cells, tissues, organs, or systems, and posing no risk of injury, toxicity, or rejection by the immune system. A biocompatible microsphere means that the microsphere, and any degradation products of the microsphere, is non-toxic to the recipient and also presents no significant deleterious or untoward effects on the recipient's body, such as an immunological reaction at the injection site.

Generally speaking, "biodegradable" means capable of being decomposed by the action of biological agents.

A biodegradable microsphere, as defined herein, means the microsphere will degrade or erode in vivo to form smaller chemical species. Degradation can result, for example, by enzymatic, chemical and/or physical processes.

Suitable biocompatible, biodegradable polymers include, for example, poly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, biodegradable polyurethanes, blends and copolymers thereof.

A number of techniques to produce microparticles have been described in the prior art.

The drug release profile for a microparticle is dependent on numerous factors, including physicochemical properties of the used polymers, interactions among polymer-drug-excipients and/or morphology and composition of the resulting microparticles.

In order for the microspheres to be retained by the myocardial capillaries they must have very specific size distribution so that their destruction through phagocytosis by macrophages, on the one hand, or embolization of the arteries, on the other, is minimised; microencapsulation using a biodegradable, biocompatible polymer enables controlled release of the product from the initial hours for up to 2-3 weeks, the main period during which post-AMI ventricular remodelling occurs.

In the context of the present invention, as the intracoronary route is preferred, the average size of these microspheres makes allowance for the size of the myocardial capillaries; this varies between 5 and 15 microns, to be retained in the cardiac region, and without particles superior to 25 microns to prevent arterial embolization.

In one embodiment of the invention, the said microspheres are presenting a diameter which does not exceed 25 µm.

In one embodiment, 50 to 100% of the microspheres are comprised in the range of 5 to 25 µm and no particles upper than 25 µm

In a preferred embodiment, 60 to 100% of the microspheres are comprised in the range of 5 to 25 µm and no particles upper than 25 µm.

In another preferred embodiment, 70 to 100% of the microspheres are comprised in the range of 5 to 25 µm and no particles upper than 25 µm.

In a more preferred embodiment, 80 to 100% of the microspheres are comprised in the range of 5 to 25 µm and no particles upper than 25 µm.

According to the invention, the average diameter of the microspheres is in the range of 5 to 15 µm.

In an alternative embodiment, the said microspheres are presenting an average diameter from 8 to 11 µm.

The microspheres according to the invention load a high amount of the said inhibitor of microRNA in a biodegradable and biocompatible polymer to sustained release the drug in the infarcted area.

The inhibitor of microRNA loading is from about 1 to 20% (w/w), preferably from 1 to 15% (w/w), and more preferably 1 to 10 and still more preferably from 5 to 10%. The drug integrity is conserved.

In one embodiment, the microspheres according to the invention are incorporating from 1% to 15 % w/w of inhibitor.

In another embodiment, the microspheres according to the invention are incorporating from 5% to 15 % w/w of inhibitor.

Still in another embodiment, the microspheres according to the invention are incorporating from 1% to 10%, preferably from 5% to 10 % w/w of inhibitor.

Another characteristic of the invention relates on the nature of the polymer used for the generation of the microspheres.

In an embodiment of the invention, the said microspheres are made of a polymer consisting of poly-d,l-lactide (PLA). In an embodiment, the said microspheres are made of PLA as the sole polymer. In an embodiment, the said microspheres are made of PLA which is blended with one or more other biocompatible polymers.

In another embodiment of the invention, the said microspheres are made of a copolymer consisting of poly-d,l-lactide-co-glycolide (PLGA). In an embodiment, the said microspheres are made of PLGA as the sole polymer. In an embodiment, the said microspheres are made of PLGA which is blended with one or more other biocompatible polymers.

Still in another embodiment of the invention, the said microspheres are made of a blend of polymers consisting of poly-d,l-lactide-co-glycolide (PLGA) and poly-d,l-lactide (PLA).

By the expression "blend", it must be understood that a mixture of two or more polymers is realized before the dissolution of the said mixture into the same organic solvent.

The man skilled in the art will easily understand that, contrary to the PLGA copolymer, the ratio of lactide:glycolide in the PLA polymer is comprised between 100:0 molar ratio.

Regarding the PLGA copolymer, the ratio of lactide:glycolide in the PLGA polymer is comprised between 50:50 to 95:5 molar ratio.

In a preferred embodiment, the ratio of lactide:glycolide in the PLGA copolymer is comprised between 50:50 to 90:10 molar ratio, and preferentially between 50:50 to 80:20 molar ratio.

In an embodiment of the invention, the inherent viscosity of the polymer is comprised between 0.1 and 0.7 dl/g.

In a preferred embodiment, the inherent viscosity of the polymer is comprised between 0.15 and 0.7 dl/g, preferentially from 0.15 to 0.5 dl/g.

This aspect is of interest in the sense that inherent viscosity is related with the polymer molecular weight and, therefore, influences in the inhibitor release rate from the polymeric microspheres.

Accordingly, it is described herein a method for producing a microencapsulated inhibitor of miR-92a which does not include any adjuvant and which consists of a single population of particles in terms of polymeric composition.

Thus, it is also described herein a method for microencapsulating an inhibitor of microRNA in polymeric microspheres, comprising: (a) dissolving the inhibitor of microRNA in purified water, without any stabilizer (b) dissolving polymer in an organic solvent; (c) adding (a) to (b) to produce a first emulsion; (d) adding the emulsion of step (c) to an aqueous solution containing a surfactant and an osmotic agent to produce a second emulsion; (e) hardening and harvesting the resulting microspheres of step (d); and (f) drying.

More particularly, it is described herein a method for producing a composition as above described, characterized in that it comprises the following steps:
a) dissolving the inhibitor of miRNA in purified water, without any stabilizer;
b) dissolving polymer in an organic solvent;
c) adding (a) to (b) to produce a first emulsion;
d) adding the emulsion of step (c) to an aqueous solution containing a surfactant and an osmotic agent to produce a second emulsion; and
e) hardening and harvesting the resulting microspheres of step (d); and
f) drying the obtained microspheres.

Another aspect of the invention consists of the use of the composition according to the invention in the treatment of the myocardial infarction.

In other word, the invention relates to a composition comprising an effective amount of at least one inhibitor of microRNA, wherein said inhibitor is microencapsulated into polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a, for use in the treatment of myocardial infarction.

In a preferred embodiment, the said myocardial infarction consists of the acute myocardial infarction.

The invention also relates to a composition comprising an effective amount of at least one inhibitor of microRNA, wherein said inhibitor is microencapsulated into polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a, for use in a method of reversing or preventing ventricular remodelling in a subject in need thereof.

Thus the invention also relates to a composition as defined above for reversing or preventing ventricular remodelling in a subject in need thereof, comprising administering to said subject an effective amount of said composition

As already mentioned, the composition according to the invention is suitable for an administration by intracoronary route.

For administration by intracoronary route, the microspheres must be suspended in an appropriate vehicle, either a saline solution (PBS) with or without a surfactant or in another appropriate vehicle for intravenous administration. Suitable dispersants include, for example, surfactants such as polysorbate 80, polysorbate 20, polyoxyethylene hydrogenated castor oil 60, carboxymethylcellulose, or polysaccharides such as sodium alginate; it is also possible add an isotonizing agent such as sodium chloride, mannitol, sorbitol or glucose, for example. Given the size of coronary arteries, the concentration of microspheres in the administration medium is adjusted in order to limit blood flow alteration and prevent the risk of arterial embolization. This concentration can vary between 0.05% and 1%, preferably between 0.1% and 0.5%. Administration can be by a single injection or repeated injections, followed optionally by a saline injection. The administration could be carried out after the percutaneous coronary angioplasty, without limitation, using the same catheter.

The method of the invention is characterized in that said administration consists of an administration by intracoronary route.

This aspect is of particular interest as it addresses several presupposed limitations of direct intravenous administration of compounds such as oligonucleotide like antagomir. Among other presupposed limitation, it can be mentioned i) low-level biosafety due to the ubiquity and low organ specificity of miRNAs, ii) high doses and repeated injections for the microRNA inhibitor to produce its effect, and iii) high theoretical cost of the calculated intravenous dose.

Surprisingly, as it will be apparent after the reading of the following examples, all these issues are addressed by the invention.

More particularly, it is demonstrated that:
a) it is possible to selectively administer the encapsulated antagomir in the artery supplying the diseased tissue (see Example 5);
b) microspheres are retained in the coronary. (see Example 6);
c) intra-arterial administration of microspheres is used for tumour embolization, enabling permanent blood flow interruption and preventing tumour progression, optionally in combination with active release substances. Taking into consideration these elements, the existence of the risk of embolization was considered. For this reason, studies were designed with the objective of ascertaining that the microspheres cause no damage to cardiac muscle or produce any significant alterations in coronary flow rate. (see Example 7);
d) good stability of the inhibitor of microRNA in the vehicle and sustained release of said inhibitor from microspheres; this is demonstrated by its biological effect while inhibiting microRNA for up to 10 days after it is administered (Example 8);
e) administration of microspheres with the miRNA inhibitor promotes contractile recovery of damaged tissue and prevents the occurrence of adverse post-infarction remodelling. (see Example 9);
f) with localised administration of microspheres, the inhibitor dose could be reduced to a single injection, which would presume a significant reduction of potential side effects and a clear reduction in cost.

The availability of an appropriate vehicle/system for the controlled administration, delivery and release of microRNA inhibitors according to the invention has the following advantages:
- Enhanced biosafety, since bio-distribution of the drug by tissue and organs that are not the treatment target, is limited
- Avoids repeated intravenous injections i) reduces hospital admissions and outpatient hospital visits, by enhancing the quality of patient support, ii) avoids the need for prolonged mainlining for drug administration as well as potential risks resulting from this and iii) minimizes the risks inherent in intravenous administration of products (infections, localised reactions ...)
- Dose reduction allows the reduction in related dose-dependent adverse effects
- Reduction of costs due to a reduction in the doses required as well as the staff and equipment required for repeated injections

In another embodiment, the invention relates to a population of biodegradable and biocompatible microspheres for use in the treatment or prevention of ventricular remodelling after myocardial infarction, wherein said microspheres:
- have an average diameter comprised between 5 and 15 µm;
- are made of poly-d,l-lactide-co-glycolide (PLGA) ; poly-d,l-lactide (PLA) or a blend thereof;
- are incorporating from 1% to 15 % w/w of a therapeutic agent capable of preventing ventricular remodelling, which includes from 1 % to 10 % w/w of said therapeutic agent,
wherein said therapeutic agent consists of an inhibitor of a microRNA consisting of miR-92a, wherein said inhibitor of microRNA is preferentially an antagomir.

The invention also relates to a kit comprising at least i) a composition and/or microspheres according to the invention and ii) a syringe or vial or ampoule in which the composition is disposed.

In an embodiment, the kit of the invention further comprises a solvent disposed in a solvent container. The solvent container may be a vial, an ampoule or a prefilled syringe

The microspheres and the solvent may be disposed in a double compartment prefilled syringe.

In an embodiment, the kit of the invention may comprise the microspheres in a vial and the solvent in a separate vial.

In an embodiment, the kit of the invention may comprise the microspheres in a vial and the solvent in a separate ampoule.

In an embodiment, the kit of the invention may comprise the microspheres in a vial and the solvent in a prefilled syringe.

In an embodiment, the kit of the invention may comprise the microspheres in a prefilled syringe and the solvent in a separate vial.

In an embodiment, the kit of the invention may comprise the microspheres in a prefilled syringe and the solvent in a separate ampoule.

In an embodiment, the kit of the invention may comprise the microspheres and the solvent separately in a double compartment syringe.

The invention will be better understood in respect to the following examples.

### Example 1: Preparation of Microspheres loaded with antagomir-92a

Microspheres were prepared by w/o/w emulsion/solvent evaporation method using a 50:50 PLGA copolymer, intrinsic viscosity of about 0.2 dL/g, which contains free carboxyl end groups. 3 ml of methylene chloride were added to 0.6 g or PLGA. 0.3 ml of a concentrated solution of Antagomir-92a (I-Ssc-miR-92a; molecular mass: 5366 g/mol; sequence: CCGGGACAAGTGCAAT; DNA Bases: 9; LNA Bases: 7; fabricant: IDT (Exiqon)) (222 mg/ml) in purified water was added to the PLGA organic solution and emulsified by sonication for 20 s. This primary emulsion was added to an external phase consisting of an aqueous solution of 1% (w/v) polyvinyl alcohol and 1% (w/v) of sodium chloride and homogenised for 60 s at about 10300 rpm. The second emulsion (w/o/w) obtained was added to a volume of purified water and the methylene chloride was allowed to evaporate by stirring. The obtained microspheres were collected by centrifugation, washed twice with purified water, and then freeze dried. Average diameter of microspheres was 9 µm, (82% between 5-25 µm and 0% upper 25 µm) while encapsulation efficiency was 74%.

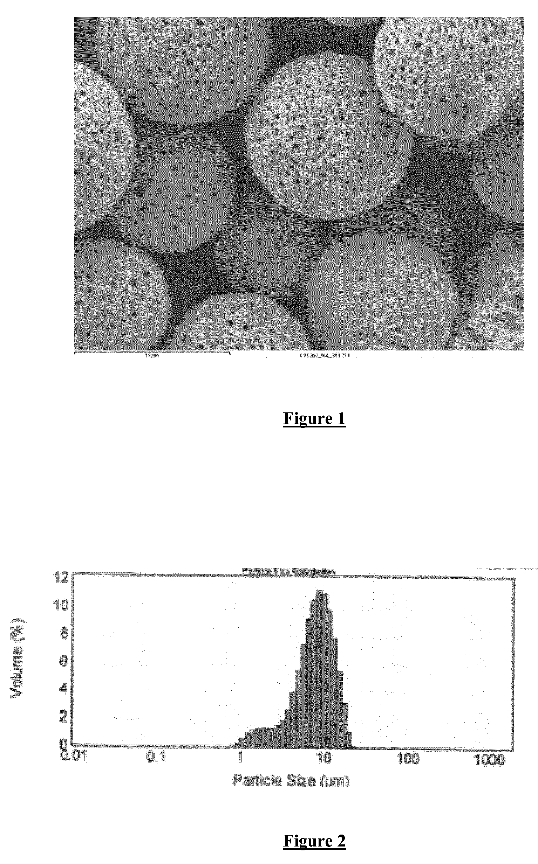

A picture of the obtained microspheres is represented in Figure 1.

Figure 2 illustrates the distribution of the microsphere size.

### Example 2: Preparation of Microspheres loaded with RNA

Microspheres were prepared by w/o/w emulsion/solvent evaporation method using a 50:50 PLGA copolymer, intrinsic viscosity of about 0.2 dL/g, which contains free carboxyl end groups. 3 ml of methylene chloride were added to 0.6 g or PLGA. 0.3 ml of a concentrated solution of RNA (222 mg/ml) (RNA Sigma 5000-10000Da) in purified water RNAsa free was added to the PLGA organic solution and emulsified by sonication for 20 s. This primary emulsion was added to an external phase consisting of an aqueous solution RNAsa free of 1% (w/v) polyvinyl alcohol and 5% (w/v) of mannitol and homogenised for 60 s at about 10300 rpm. The second emulsion (w/o/w) obtained was added to a volume of purified water RNAsa free and the methylene chloride was allowed to evaporate by stirring. The obtained microspheres were collected by centrifugation, washed twice with purified water RNAsa free, and then freeze dried. Average diameter of microspheres was 10 µm, (86% between 5-25 µm and 0% upper 25 µm) while encapsulation efficiency was 73%.

### Example 3: Preparation of placebo Microspheres

Microspheres were prepared by w/o/w emulsion/solvent evaporation method using a 50:50 PLGA copolymer, intrinsic viscosity of about 0.2 dL/g, which contains free carboxyl end groups. 3 ml of methylene chloride were added to 0.6 g or PLGA. 0.3 ml of purified water was added to the PLGA organic solution and emulsified by sonication for 20 s. This primary emulsion was added to an external phase consisting of an aqueous solution of 1% (w/v) polyvinyl alcohol and 1% (w/v) of sodium chloride and homogenised for 60 s at about 10300 rpm. The second emulsion (w/o/w) obtained was added to a volume of purified water and the methylene chloride was allowed to evaporate by stirring. The obtained microspheres were collected by centrifugation, washed twice with purified water, and then freeze dried. Average diameter of microspheres was 7 µm, (84% between 5-25 µm and 0% upper 25 µm).

### Example 4: Preparation of Microspheres loaded with albumin fluorescein isothiocyanate

Microspheres were prepared by w/o/w emulsion/solvent evaporation method using a 50:50 PLGA copolymer, intrinsic viscosity of about 0.2 dl/g, which contains free carboxyl end groups. 1 ml of methylene chloride was added to 0.2 g or PLGA. 0.1 ml of an albumin fluorescein isothiocyanate aqueous solution (20 mg/ml) was added to the PLGA organic solution and emulsified by sonication for 15 s. This primary emulsion was added to an external phase consisting of an aqueous solution of 1% (w/v) polyvinyl alcohol and 1% (w/v) and homogenised for 60 s at about 10300 rpm. The second emulsion (w/o/w) obtained was added to a volume of purified water and the methylene chloride was allowed to evaporate by stirring. The obtained microspheres were collected by centrifugation, washed twice with purified water, and then freeze dried. Average diameter of microspheres was 9 µm, (91% between 5-25 µm and 0% upper 25 µm).

### Example 5: Study of selective administration in the artery supplying the target tissue

After triggering an AMI in a Large White pig, 30 mg of microspheres containing fluorescent albumin, prepared as indicated in example 4, were administered by intra-coronary route, by means of a 2.5/12 coaxial balloon positioned in the artery responsible for the AMI, which supplies the infarcted area. The microspheres were suspended *in situ* in 10 ml of normal saline solution containing Tween-80; administration was performed in 2 consecutive injections of 5 ml, each followed by 5 ml of normal saline solution. Experiments showed that the encapsulated antagomir can be selectively administered in the artery supplying the diseased tissue.

### Example 6: Study of retention of the microspheres in the capillaries of the diseased tissue without allowing outflow into the bloodstream

On a pig model, 4 experiments were conducted by administering by intra-coronary route through a coaxial balloon positioned in the medial anterior descending branch, 2 injections with 5 ml each of fluorescent microspheres prepared according to example 4. The 4 animals were euthanized and myocardial samples of the tissue adjoining the anterior descending branch and the control tissue irrigated by other coronary arteries were obtained. The samples were observed through an optical fluorescence microscope and the presence of microspheres retained in the capillaries of the damaged cardiac muscle as well as their absence in the control tissue was demonstrated.

To rule out systemic biodistribution, in two of the previous animals, besides ischemic and control myocardial tissue, 5 replicate samples from lung, spleen and liver were obtained and visualized by optical microscopy with light B. Fluorescence was exclusively detected in anterior myocardial wall. This analysis revealed that microspheres are retained in the heart avoiding systemic release of antagomir92a (reduction of side effects).

### Example 7: Study of retention of microspheres in the capillaries of diseased tissue without damaging, the target tissue itself

Experiments were conducted to investigate the potential local heart toxicity and the therapeutic safety range of dose. To detect local ischemic damage to the cardiac muscle 2 paired piezoelectric crystals, which are highly sensitive in their ability to detect ischemia were employed. When cardiac muscle tissue is affected by ischemia, the remaining tissue becomes dyskinetic and swells; this, along with the blood pressure produced by the remaining contiguous healthy tissue causes the microcrystals to separate and move further away from each other. In two pigs after performing a thoracotomy and a pericardiectomy two pairs of microcrystals were inserted, one control pair in the lateral region and one pair in the anterior region supplied by the anterior descending branch, through which the microspheres were administrated. For each pair of medium crystals the distance between them at two points during the cardiac cycle was measured: at end-diastole **(EDL)** and end-systole **(ESL).** The relation between EDL and ESL is expressed by the parameter **SS** (systolic shortening: (EDL - ESL) / EDL. When the left ventricular contraction is completely dissipated EDL = ESL and SS = 0. The normal values range between 0.2 ± 0.1. As shown in the attached illustration, minimal and transient oscillations after each injection lasting a few seconds were induced with the dose of the study, corresponding to the first and second injections. Furthermore and surprisingly, no local side effects were observed with repeated intracoronary injections of fluorescent microspheres prepared according to example 4 reaching 14 times the dose of the study. No limiting maximum dose was associated with irreversible ischemic damage, hemodynamic repercussion or arrhythmias.

In addition, to detect changes in coronary flow a flow sensor was positioned in middle LAD measuring coronary flow. No significant changes in coronary flow were observed after intracoronary injections.

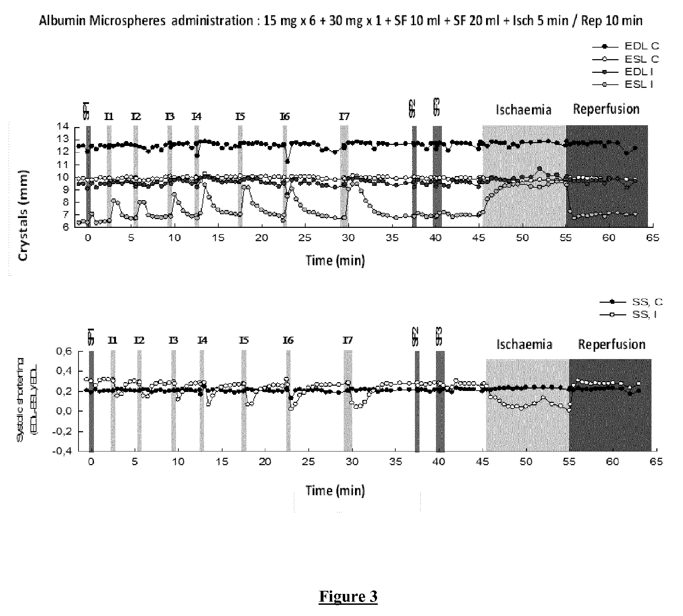

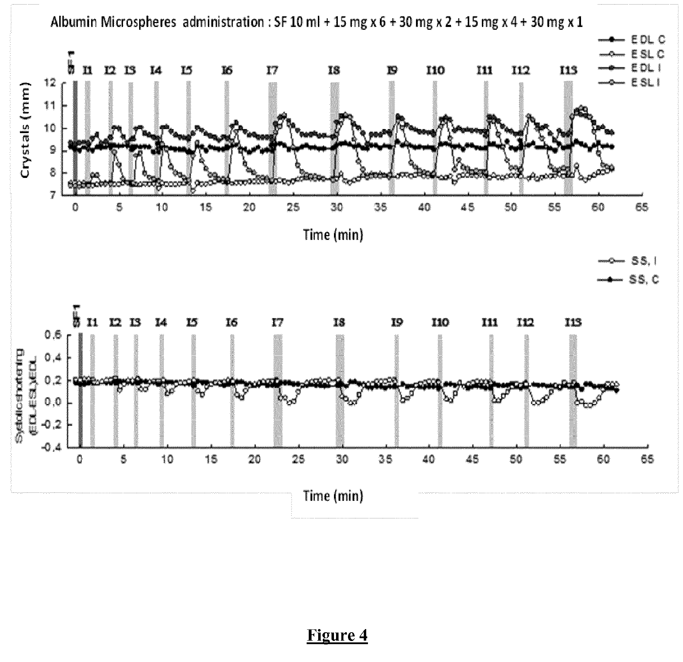

Results are illustrated by Figure 3 wherein 120mg of microspheres were injected and by Figure 4 wherein 240 mg of micropsheres were injected.

### Example 8: Study of the molecular effect of a single intra-coronary injection of microspheres with a small antagomir dose

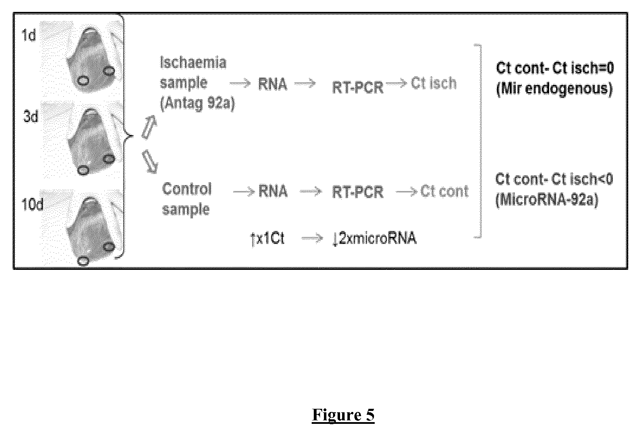

In order to demonstrate that small doses of microencapsulated antagomir could produce a molecular response, the miR-92a expression in vivo, in ischemic and control tissue, was measured after intracoronary encapsulated antagomir-92a administration. In 3 pigs, 60 mg of microspheres containing antagomir-92a prepared according to example 1 (0.1 mg/Kg) were delivered in LAD. Animals were euthanized at one, three and 10 days after treatment and expression of miR-92a and endogenous microRNA as controls (miR-123, 203, and 126) were quantified in 2 replicate infarcted and control samples by total RNA isolation and real-time quantitative RT-PCR using specific primers (see Figure 5).

In infarcted tissue, miR-92a expression resulted down-regulated by 8-fold in comparison with control tissue whereas expression of endogenous miRs was not affected by the treatment. The inhibition began to be present as early as 1 day and it was still present at day 10, with 5 times lower expression levels than in the control area.

No significant regulation was detected in endogenous miRs. These results reveals that the vehicle/system provides adequate conditions for enabling controlled delivery and release of antagomir-92a, thereby producing a sustained inhibition of microRNA-92a with a single intracoronary administration.

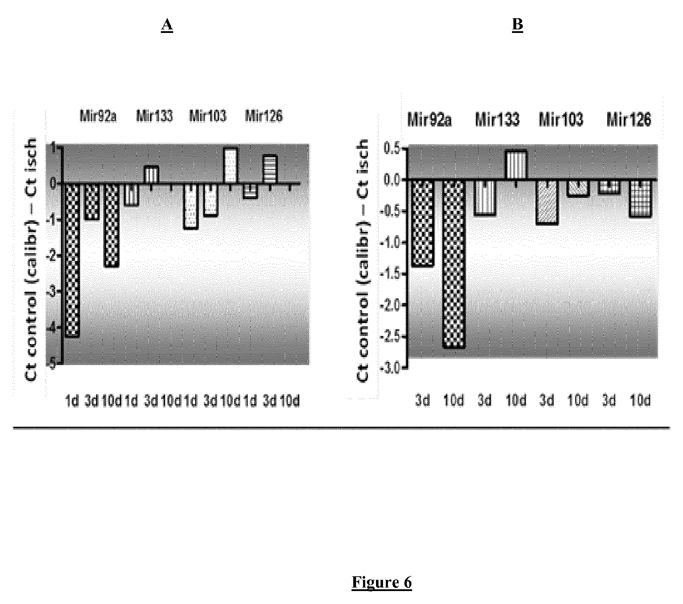

These results, represented in Figure 6, also confirm that the antagomir is not degraded during the microsphere manufacturing process.

### Example 9: Study of the biological effect of a single intra-coronary injection of microsphere containing low doses of antagomir

In order to demonstrate whether the molecular effect of microsphere-transported antagomir-92a is accompanied by a biological effect, a pre-clinical study was conducted with 26 adult minipigs. The purpose of this study was to investigate whether inhibition of mir-92a by selective intracoronary encapsulated antagomir-92a administration leads to enhance of angiogenesis in infarcted area, and thus preventing the occurrence of ventricular remodelling.

3 formulations were administered:
- Saline solution (control formulation)
- Placebo microspheres prepared according to example 3
- Antagomir-92a microspheres prepared according to example 1, at one antagomir dose of 3 mg /minipig.

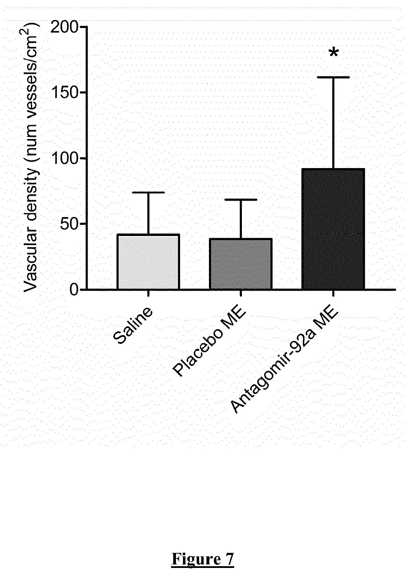

4 weeks after treatment, significant higher vascular density in the necrotic area was detected in those animals receiving encapsulated antagomir-92a compared to controls, thereby confirming the proangiogenic activity of antagomir-92a observed in previous study (161.57±58.71 vs 68.49±23.56 in placebo group vs 73.91±24.97 in saline group, p=0.001) ii) the vascular density (see Figure 7).

Microvascularity increased within both, the infarct zone and the peri-infarct rim. Lower microvascular resistance index in those treated animals was consistently demonstrated (200.67±104.46 vs 511.73±202.1 in controls, p=0.007) and it was significantly correlated with vascular density (R² 0.41, p=0.02). (see Figure 8).

These data indicate that encapsulated antagomir-92a induces sustained angiogenesis in vivo.

Having found growth vessels, its potential benefits in healing process that occurs after an AMI was therefore further investigated. To determine the effects of encapsulated antagomir-92a on ventricular remodelling, morphological and structural parameters by ex vivo magnetic resonance imaging (CMR) and functional parameters analysed by intravascular echocardiography (IVE), in the treated and non-treated groups were compared. Significant more percentage of animals with anterior and septoapical dyskinesia was present in IVE in controls (p=0.03) (see notably Figure 9) with also significant higher thinning of the injured ventricular wall and adverse remodelling morphometry changes in left ventricle in ex-vivo CMR in comparison with treated animals (Table 3).

More particularly, Figure 9 illustrates the results of the analysis of regional wall motion dysfunction by intravascular echocardiography (IVE). IVE was performed by using a Vivid Q ultrasound imaging machine (GE Healthcare, Belford, UK) and an AcuNav 10F ultrasound catheter (Siemens) placed in the apex of the right ventricle.

The results of the study show that the administration of antagomir-92a microspheres is associated with a statistically significant reduction in adverse remodelling following acute myocardial infarction.

**Table 3: Parameters of left ventricular remodelling in CMR**

| | Saline (N=6) | Placebo ME (N=5) | Antagomir-92a ME (N=6) | P |
|---|---|---|---|---|
| Number of infarctedCMR slices | 4.8±0.3 | 4.8±0.4 | 5.3±0.2 | 0.38 |
| T_{maxinfarctedwall}, mm | 6.07±0.9 | 5.61±0.5 | 9.01±0.6 | 0.006 |
| T_{nonnal posterior wall}, mm | 13.23±0.5 | 13.52±1.8 | 11.82±0.7 | 0.49 |
| Percentage of minimumthinning, % | 54.79±4.9 | 56.74±4.1 | 22.71±5.5 | 0.000 |
| T_{mininfarctedwall}, mm | 3.17±0.4 | 4.02±0.9 | 4.35±0.5 | 0.33 |
| Percentage of maximumthinning, % | 76.40±2.1 8 | 69.86±4.72 | 62.54±4.19 | 0.05 |
| Length of the thinning wall, mm | 32.2±1.8 | 31.7±4 | 20.5±3.6 | 0.03 |
| D_{R}/D_{N} | 1.93±0.2 | 2.02±0.2 | 1.29±0.1 | 0.03 |
| D_{N},mm | 14.88±0.6 8 | 13.78±1.59 | 17.5±1.37 | 0.12 |
| Adverse remodeling% (n) | 83.3 (5) | 80 (4) | 16.7 (1) | 0.03 |

Encapsulated antagomir-92a prevents adverse left ventricular remodelling 1 month after acute myocardial infarction. The different remodelling parameters calculated in all infarcted slices of ex-vivo CMR in each minipig were dtermined. Representative L2 slice (being L1 the apex) of four minipigs are shown.T_{max infarcted wall} = mean maximum infarcted wall thickness calculated as Σ of the maximum infarct wall thickness in each slice divided by the number of affected slices; Tₙₒᵣₘₐₗ posterior _{wall} = mean thickness of normal posterior wall measured just beside the insertion of posterior papillary muscle calculated as Σ of the wall posterior thickness in each affected slice divided by de number of affected slices; mean percentage of minimum thinning calculated as [100 - (T_{max infarcted wall}/T ₙₒᵣₘₐₗ posterior wₐₗₗ x100)];Tₘᵢₙ infarcted _{wall} = mean minimum infarcted wall thickness calculated as Σ of the minimum infarct wall thickness in each affected slice divided by the number of affected slices; mean percentage of maximum thinning calculated as [100 - (Tₘᵢₙ infarcted _{wall}/T _{normal posterior wall}x100)]; D_{R}: mean maximal diameter between infarcted wall and contralateral normal wall calculated as ∑ of the maximal diameter between infarcted wall in each infarcted slice divided by the number of affected slices; D_{N:} mean maximal diameter between normal walls, forming a right angle with D_{R} and drawn nearest thecenterofthe ventricularcavity, calculated as ∑ of the maximal diameter between normal walls in each infarcted slice divided by the number of affected slices; D_{R}/ D_{N:} mean sphericity index calculated as ∑ of the D_{R}/ D_{N} of each infarcted slice divided by the number of affected slices. Data of the table are expressed as the mean ± s.e.m.

The results of a epresentative CMR show that:
*A: Cardiac NMR and IVE of minipig 14 (death immediately after induction of AMI):* Owing to the occurrence of death immediately following the AMI, there was not enough time for the remodelling process to be triggered. This is why a concentric left ventricle was seen to have similar dimensions in all of the segments.
*B: Cardiac NMR and IVE of the minipig 20 to 30 days post-AMI: evidence of adverse ventricular remodelling:* One month after the AMI, extreme emaciation of the anterior and septal sections was observed on the CMR as well as an aneurysm formation with dyskinesia on the IVE which is typical of post-AMI adverse remodelling.
*C: Cardiac NMR and IVE of the minipig 22 to 30 days post-AMI: no ventricular remodelling:* One month after AMI, slight reduction of the parietal region in the anterior and septal areas was observed without aneurysm formation and without dyskinesia in the IVE. This is a typical case of favourable repair reactions after AMI.

### Example 10: Study of the induction of vascular tumours or effects in short-term mortality of encapsulated antagomir-92a

No vascular tumors were observed in necropsy analysis performed to all animals, thereby suggesting the absence of ectopic systemic suppression of microRNA-92a in other organs at distance. The mortality of the study was of 23 %. No differences were observed in short-term mortality. Only one minipig allocated to encapsulated antagomir92a died (p=0.39).

**Table 4**

| **N=26** | **Saline (n=9)** | **Placebo ME (n=9)** | **Antagomir-92a ME (n=8)** |
|---|---|---|---|
| **1 month follow-up** | **6** | 7 | 7 |
| **Death** | **3** | **2** | **1** |

### Example 11: Study of the proarrhythmic profile of encapsulated antagomir-92a.

In order to know the arrhythmogenic potential of encapsulated antagomir-92a, all arrhythmic events during the procedures were recorder and analyzed by Collect 5S software (GE). Moreover, to address this issue, an insertable loop recorder was randomly implanted in 10 of the 26 minipigs of the study to detect potential episodes of arrhythmia until sacrifice, at one month postinfarction and treatment. No higher number of maligne tachyarrhythmias nor bradiarrhythmias were observed in treated group in comparison with controls, indicating that intracoronary encapsulated antagomir-92a doesn't exert a proarrhythmic effect.

**Table 5: Arrhythmias detected during the study**

| | Saline (n=9) | Placebo ME (n=9) | Antagomir-92a ME (n=8) | p |
|---|---|---|---|---|
| *Ischemic phase (n= 26)* | | | | |
| No arrhythmias, n(%) | 2 (22.2) | 0 | 1 (12.5) | 0.37 |
| Arrhythmias, n(%) | 7 (77.8) | (100) | 7 (87.5) | |
| PVC, n | 5 | 7 | 5 | |
| NSVT, n | 0 | 1 | 1 | |
| Ventricular fibrilation, n | 3 | 3 | 3 | |

| *Reperfusion phase (n=26)* | | | | |
|---|---|---|---|---|
| No arrhythmias, n(%) | 5 (55.6) | 3 (33.3) | 3 (37.5) | 0.6 |
| Arrhythmias, n(%) | 4 (44.4) | 6 (66.7) | 5 (62.5) | |
| Sinusal pauses, n | 1 | 1 | 0 | |
| Nodal rhythm,n | 1 | 0 | 0 | |
| IVR, n | 0 | 2 | 1 | |
| PVC, n | 4 | 3 | 3 | |
| NSVT, n | 0 | 0 | 1 | |

| *During 30 days after AMI (n=10)* | | | | |
|---|---|---|---|---|
| *Implantable loop recorder* | n=4 | n=3 | n=3 | 0.88 |
| No arrhythmias, n(%) | 0(0) | 0(0) | 0(0) | 0.38 |
| Arrhythmias, n(%) | 2(50) | 3 (100) | 3 (100) | |
| Sinusal taquicadia | 2 | 3 | 3 | |
| Sinusal pausa | 0 | 1 | 0 | |
| PVC or PSVC | 0 | 0 | 1 | |
| Not assessable | 2(50) | 0 | 0 | |

| | | | | |
|---|---|---|---|---|
| PVC: premature ventricular complexes, NSVT: non-sustained ventricular taquicardia, IVR: idioventricular rhythm, PSVC: premature supraventricular complexes AMI: acute myocardial infarction | | | | |

## Claims

1. Composition comprising an effective amount of at least one inhibitor of a miRNA involved in the angiogenesis, wherein said inhibitor is microencapsulated into polymeric biodegradable and biocompatible microspheres, wherein at least 50% of said microspheres are presenting a diameter comprised between 5 and 15 µm, and wherein the said at least one inhibitor consists of an antisense oligonucleotide targeting or being complementary to miR-92a.

2. The composition of claim 1, wherein said miRNA consists of the mature miR-92a comprising a sequence selected from the group consisting of SEQ ID No. 21, 22 or 23 or a sequence having at least 90% nucleotide identity with one of SEQ ID No. 21, 22 or 23.

3. The composition of claim 1, wherein the said antisense oligonucleotide is an oligonucleotide of 8-49 nucleotides in length.

4. The composition of claim 1, wherein the said antisense oligonucleotide is at least partially complementary to the sequence of the target miRNA.

5. The composition of claim 1, wherein said antisense oligonucleotide is selected from the group consisting of a ribonucleotide, a deoxyribonucleotide, an antagomir, a LNA, a PNA, a morpholino oligonucleotide or a combination thereof.

6. The composition of claim 5, wherein said antisense oligonucleotide is an antagomir.

7. The composition of claim 6, wherein said antagomir comprises a nucleotide sequence comprising at least 16 contiguous nucleotides complementary to the nucleotides of a sequence selected from the group consisting of SEQ ID No. 1 and 2.

8. The composition of claim 7, wherein said antagomir comprises the sequence SEQ ID No. 59 or 60 and modifications excluding base substitutions thereof, and fragments consisting of subsequences of SEQ ID NO: 59 or 60 of at least 8 contiguous nucleotides thereof.

9. The composition of claims 1 to 8, wherein said microspheres are presenting a diameter which does not exceed 25 µm.

10. The composition of claims 1 to 9, wherein the microspheres are incorporating from 1% to 15 % w/w of inhibitor.

11. The composition of claims 1 to 9, wherein the microspheres are incorporating from 1% to 10 % w/w of inhibitor.

12. The composition of claims 1 to 11, wherein said microspheres are made of a polymer consisting of poly-d,l-lactide (PLA), the said polymer being optionally blended with one or more other polymers.

13. The composition of claims 1 to 11, wherein said microspheres are made of a copolymer consisting of poly-d,l-lactide-co-glycolide (PLGA), the said polymer being optionally blended with one or more other polymers.

14. The composition of claims 1 to 11, wherein said microspheres are made of a blend of polymers consisting of poly-d,1-lactide-co-glycolide (PLGA) and poly-d,l-lactide (PLA).

15. The composition of claims 13 or 14, wherein the ratio af lactide:glycolide in the PLGA polymer is comprised between 50:50 to 95:5 molar ratio.

16. The composition of claims 13 to 15, wherein the inherent viscosity of the polymer is comprised between 0.1 and 0.70 dl/g.

17. The composition of any one of the preceding claims for use in the treatment of the myocardial infarction.

18. The composition for use according to claim 17 wherein said myocardial infarction consists of the acute myocardial infarction.

19. The composition of claims 1 to 18 for use in the reversing or preventing ventricular remodelling in a subject in need thereof.

20. A population of biodegradable and biocompatible microspheres for use in the treatment or prevention of myocardial infarction, wherein said microspheres:
- have an average diameter comprised between 5 and 15 µm;
- are made of poly-d,l-lactide-co-glycolide (PLGA) ; poly-d,l-lactide (PLA) or a blend thereof;
- are incorporating from 1% to 15% w/w of a therapeutic agent capable of preventing ventricular remodelling,
wherein said therapeutic agent consists of an antisense inhibitor of a miRNA consisting of miR-92a.

21. The microspheres for use according to claim 20, wherein said inhibitor is an antagomir.

22. A kit comprising at least i) a composition according to claim 1 to 16 and/or microspheres according to claims 20 or 21 and ii) a syringe or vial or ampoule in which the composition is disposed.

23. The kit of claim 22, further comprising a solvent disposed in a solvent container.

## Patentansprüche

1. Zusammensetzung, die eine wirksame Menge mindestens eines Inhibitors einer miRNA umfasst, die in der Angiogenese beteiligt ist, wobei der Inhibitor in polymeren bioabbaubaren und biokompatiblen Mikrokügelchen mikroverkapselt ist, wobei mindestens 50% der Mikrokügelchen einen Durchmesser aufweisen, der zwischen 5 und 15 µm liegt, und wobei der mindestens eine Inhibitor aus einem Antisense-Oligonukleotid besteht, das zu miR-92a zielgerichtet bzw. komplementär ist.

2. Zusammensetzung nach Anspruch 1, wobei die miRNA aus maturer miR-92a besteht, die eine Sequenz, die aus der aus SEQ ID Nr. 21, 22 oder 23 bestehenden Gruppe ausgewählt ist, oder eine Sequenz mit mindestens 90% Nukleotid-Identität mit SEQ ID Nr. 21, 22 oder 23 umfasst.

3. Zusammensetzung nach Anspruch 1, wobei das Antisense-Oligonukleotid ein Oligonukleotid von 8 bis 39 Nukleotiden Länge ist.

4. Zusammensetzung nach Anspruch 1, wobei das Antisense-Oligonukleotid mindestens teilweise komplementär zu der Sequenz der Ziel-miRNA ist.

5. Zusammensetzung nach Anspruch 1, wobei das Antisense-Oligonukleotid aus der aus Ribonukleotid, Desoxyribonukleotid, Antagomir, LNA, PNA, Morpholino-Oligonukleotid oder einer Kombination daraus bestehenden Gruppe ausgewählt ist.

6. Zusammensetzung nach Anspruch 5, wobei das Antisense-Oligonukleotid ein Antagomir ist.

7. Zusammensetzung nach Anspruch 6, wobei das Antagomir eine Nukleotidsequenz mit mindestens 16 zusammenhängenden Nukleotiden umfasst, die komplementär zu den Nukleotiden einer Sequenz sind, die aus der aus SEQ ID No. 1 und 2 bestehenden Gruppe ausgewählt ist.

8. Zusammensetzung nach Anspruch 7, wobei das Antagomir die Sequenz SEQ ID Nr. 59 oder 60 und deren Modifikationen mit Ausnahme von Basensubstitutionen und Fragmente aus Teil-sequenzen von SEQ ID Nr. 59 oder 60 aus mindestens acht zusammenhängenden Nukleotiden daraus aufweist.

9. Zusammensetzung nach einem der Ansprüche 1 bis 8, wobei die Mikrokügelchen einen Durchmesser aufweisen, der 25 µm nicht übersteigt.

10. Zusammensetzung nach einem der Ansprüche 1 bis 9, wobei die Mikrokügelchen Gewicht/Gewicht 1% bis 15% Inhibitor beinhalten.

11. Zusammensetzung nach einem der Ansprüche 1 bis 9, wobei die Mikrokügelchen Gewicht/Gewicht 1% bis 10% Inhibitor beinhalten.

12. Zusammensetzung nach einem der Ansprüche 1 bis 11, wobei die Mikrokügelchen aus einem Polymer hergestellt sind, das aus Poly-d,1-Lactid (PLA) besteht und optional mit einem oder mehreren anderen Polymeren gemischt ist.

13. Zusammensetzung nach einem der Ansprüche 1 bis 11, wobei die Mikrokügelchen aus einem Copolymer hergestellt sind, das aus Poly-d,1-Lactid-co-Glycolid (PLGA) besteht und optional mit einem oder mehreren anderen Polymeren gemischt ist.

14. Zusammensetzung nach einem der Ansprüche 1 bis 11, wobei die Mikrokügelchen aus einer Mischung aus Polymeren hergestellt sind, die aus Poly-d,1-Lactid-co-Glycolid (PLGA) und Poly-d,1-Lactid (PLA) bestehen.

15. Zusammensetzung nach Anspruch 13 oder 14, wobei das Lactid:Glycolid-Verhältnis im PLGA-Polymer ein Molverhältnis von 50:50 bis 95:5 aufweist.

16. Zusammensetzung nach einem der Ansprüche 13 bis 15, wobei die inhärente Viskosität des Polymers zwischen 0,1 und 0,70 dl/g beträgt.

17. Zusammensetzung nach einem der vorhergehenden Ansprüche zur Verwendung in der Behandlung des Myokardinfarkts.

18. Zusammensetzung zur Verwendung nach Anspruch 17, wobei der Myokardinfarkt der akute Myokardinfarkt ist.

19. Zusammensetzung nach einem der Ansprüche 1 bis 18 zur Verwendung in der Reversion oder Prävention ventrikulärer Umbildung in einem Geschöpf, das dies benötigt.

20. Anzahl bioabbaubarer und biokompatibler Mikrokügelchen zur Verwendung in der Behandlung oder Prävention von Myokardinfarkt, wobei die Mikrokügelchen:
einen mittleren Durchmesser zwischen 5 und 15 µm aufweisen;
aus Poly-d,1-Lactid-co-Glycolid (PLGA); Poly-d,1-Lactid (PLA) oder einer Mischung daraus hergestellt sind;
von 1% bis 15% Gewicht/Gewicht eines therapeutischen Mittels beinhalten, das ventrikulärer Umbildung vorbeugen kann,
wobei das therapeutische Mittel aus einem Antisense-Inhibitor einer miRNA aus miR-92a besteht.

21. Mikrokügelchen zur Verwendung nach Anspruch 20, wobei der Inhibitor ein Antagomir ist,

22. Kit mit mindestens i) einer Zusammensetzung nach einem der Ansprüche 1 bis 16 und/oder Mikrokügelchen nach Anspruch 20 oder 21 sowie ii) einer Spritze oder Phiole oder Ampulle, in die die Zusammensetzung gegeben ist.

23. Kit nach Anspruch 22 mit einem Lösungsmittel, das in einen Lösungsmittelbehälter gegeben ist.

## Revendications

1. Composition comprenant une quantité efficace d'au moins un inhibiteur d'un miARN impliqué dans l'angiogenèse, dans laquelle ledit inhibiteur est microencapsulé dans des microsphères polymères biodégradables et biocompatibles, dans laquelle au moins 50 % desdites microsphères présentent un diamètre compris entre 5 et 15 µm, et dans laquelle ledit au moins un inhibiteur consiste en un oligonucléotide antisens ciblant ou étant complémentaire à miR-92a.

2. Composition selon la revendication 1, dans laquelle ledit miARN consiste en le miR-92a mature comprenant une séquence sélectionnée dans le groupe consistant en SEQ ID NO: 21, 22 ou 23 ou une séquence présentant au moins 90 % d'identité de nucléotides avec l'une de SEQ ID NO: 21, 22 ou 23.

3. Composition selon la revendication 1, dans laquelle ledit oligonucléotide antisens est un oligonucléotide d'une longueur de 8-49 nucléotides.

4. Composition selon la revendication 1, dans laquelle ledit oligonucléotide antisens est au moins partiellement complémentaire à la séquence du miARN cible.

5. Composition selon la revendication 1, dans laquelle ledit oligonucléotide antisens est sélectionné dans le groupe consistant en un ribonucléotide, un désoxyribonucléotide, un antagomir, un LNA, un PNA, un oligonucléotide morpholino ou une combinaison de ceux-ci.

6. Composition selon la revendication 5, dans laquelle ledit oligonucléotide antisens est un antagomir.

7. Composition selon la revendication 6, dans laquelle ledit antagomir comprend une séquence de nucléotides comprenant au moins 16 nucléotides contigus complémentaires aux nucléotides d'une séquence sélectionnée dans le groupe consistant en SEQ ID NO: 1 et 2.

8. Composition selon la revendication 7, dans laquelle ledit antagomir comprend la séquence SEQ ID NO: 59 ou 60 et des modifications excluant des substitutions de bases de celle-ci, et des fragments consistant en des sous-séquences de SEQ ID NO: 59 ou 60 d'au moins 8 nucléotides contigus de celles-ci.

9. Composition selon les revendications 1 à 8, dans laquelle lesdites microsphères présentent un diamètre qui n'excède pas 25 µm.

10. Composition selon les revendications 1 à 9, dans laquelle les microsphères incorporent entre 1 % et 15 % p/p d'inhibiteur.

11. Composition selon les revendications 1 à 9, dans laquelle les microsphères incorporent entre 1 % et 10 % p/p d'inhibiteur.

12. Composition selon les revendications 1 à 11, dans laquelle lesdites microsphères sont constituées d'un polymère consistant en le poly(d,l-lactide) (PLA), ledit polymère étant facultativement mélangé avec un ou plusieurs autres polymères.

13. Composition selon les revendications 1 à 11, dans laquelle lesdites microsphères sont constituées d'un copolymère consistant en le poly(d,l-lactide-co-glycolide) (PLGA), ledit polymère étant facultativement mélangé avec un ou plusieurs autres polymères.

14. Composition selon les revendications 1 à 11, dans laquelle lesdites microsphères sont constituées d'un mélange de polymères consistant en le poly(d,l-lactide-co-glycolide) (PLGA) et le poly(d,l-lactide) (PLA).

15. Composition selon les revendications 13 ou 14, dans laquelle le rapport de lactide:glycolide dans le polymère PLGA est un rapport molaire compris entre 50:50 et 95:5.

16. Composition selon les revendications 13 à 15, dans laquelle la viscosité inhérente du polymère est comprise entre 0,1 et 0,70 dl/g.

17. Composition selon l'une quelconque des revendications précédentes destinée à être utilisée dans le traitement de l'infarctus du myocarde.

18. Composition destinée à être utilisée selon la revendication 17, où ledit infarctus du myocarde consiste en l'infarctus aigu du myocarde.

19. Composition selon les revendications 1 à 18 destinée à être utilisée pour inverser ou prévenir le remodelage ventriculaire chez un sujet en ayant besoin.

20. Population de microsphères biodégradables et biocompatibles destinée à être utilisée dans le traitement ou la prévention de l'infarctus du myocarde, dans laquelle lesdites microsphères :
- possèdent un diamètre moyen compris entre 5 et 15 µm ;
- sont constituées de poly(d,l-lactide-co-glycolide) (PLGA) ; poly(d,l-lactide) (PLA) ou d'un mélange de ceux-ci ;
- incorporent entre 1 % et 15 % p/p d'un agent thérapeutique capable de prévenir le remodelage ventriculaire,
où ledit agent thérapeutique consiste en un inhibiteur antisens d'un miARN consistant en miR-92a.

21. Microsphères destinées à être utilisées selon la revendication 20, dans lesquelles ledit inhibiteur est un antagomir.

22. Kit comprenant au moins i) une composition selon la revendication 1 à 16 et/ou des microsphères selon les revendications 20 ou 21 et ii) une seringue ou un flacon ou une ampoule dans laquelle/lequel la composition est disposée.

23. Kit selon la revendication 22, comprenant en outre un solvant disposé dans un récipient de solvant.
